(19) Europäisches Patentamt
European Patent Office
Office européen des brevets

(11) EP 3 457 928 B1

(12) **EUROPEAN PATENT SPECIFICATION**

(45) Date of publication and mention of the grant of the patent:
**20.11.2019 Bulletin 2019/47**

(21) Application number: **17726891.9**

(22) Date of filing: **22.05.2017**

(51) Int Cl.:
***A61B 8/08*** (2006.01) ***A61B 5/00*** (2006.01)
***A61B 5/02*** (2006.01) ***A61B 5/021*** (2006.01)
***A61B 5/0215*** (2006.01) ***A61B 5/20*** (2006.01)
***A61B 5/0285*** (2006.01) ***A61B 8/04*** (2006.01)
***A61B 8/06*** (2006.01) *A61B 5/022* (2006.01)

(86) International application number:
**PCT/EP2017/062230**

(87) International publication number:
**WO 2017/198871 (23.11.2017 Gazette 2017/47)**

(54) **DETERMINING PULSE WAVE VELOCITY USING INTRAVASCULAR PRESSURE MEASUREMENT AND EXTERNAL ULTRASOUND IMAGING, AND ASSOCIATED DEVICES, SYSTEMS, AND METHODS**

BESTIMMUNG DER PULSWELLENGESCHWINDIGKEIT MITTELS MESSUNG DES INTRAVASKULÄREN DRUCKS UND EXTERNER ULTRASCHALLBILDGEBUNG, SOWIE ZUGEHÖRIGE VORRICHTUNGEN, SYSTEME UND VERFAHREN

DÉTERMINATION DE LA VITESSE DES ONDES DE PRESSION ET DE MESURE DE PRESSION INTRAVASCULAIRE UTILISANT UNE IMAGERIE ULTRASONORE EXTERNE, ET DISPOSITIFS , SYSTÈMES, ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2016 EP 16170635**
**29.06.2016 EP 16176863**

(43) Date of publication of application:
**27.03.2019 Bulletin 2019/13**

(73) Proprietor: **Koninklijke Philips N.V.**
**5656 AE Eindhoven (NL)**

(72) Inventors:
- **VAN DER HORST, Arjen**
**5656 AE Eindhoven (NL)**
- **SIO, Charles, Frederik**
**5656 AE Eindhoven (NL)**
- **KUENEN, Maarten, Petrus, Joseph**
**5656 AE Eindhoven (NL)**

(74) Representative: **Philips Intellectual Property & Standards**
**High Tech Campus 5**
**5656 AE Eindhoven (NL)**

(56) References cited:
**WO-A2-00/55579**
**US-A1- 2006 058 653**
**US-A1- 2009 270 695**
**US-A1- 2014 012 133**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 457 928 B1

## Description

### TECHNICAL FIELD OF THE INVENTION

Embodiments of the present disclosure relate generally to the field of medical devices and, more particularly, to apparatuses, systems, and methods for determining pulse wave velocity.

### BACKGROUND OF THE INVENTION

Hypertension and its associated conditions, chronic heart failure (CHF) and chronic renal failure (CRF), constitute a significant and growing global health concern. Current therapies for these conditions span the gamut covering non-pharmacological, pharmacological, surgical, and implanted device-based approaches. Despite the vast array of therapeutic options, the control of blood pressure and the efforts to prevent the progression of heart failure and chronic kidney disease remain unsatisfactory.

Blood pressure is controlled by a complex interaction of electrical, mechanical, and hormonal forces in the body. The main electrical component of blood pressure control is the sympathetic nervous system (SNS), a part of the body's autonomic nervous system, which operates without conscious control. The sympathetic nervous system connects the brain, the heart, the kidneys, and the peripheral blood vessels, each of which plays an important role in the regulation of the body's blood pressure. The brain plays primarily an electrical role, processing inputs and sending signals to the rest of the SNS. The heart plays a largely mechanical role, raising blood pressure by beating faster and harder, and lowering blood pressure by beating slower and less forcefully. The blood vessels also play a mechanical role, influencing blood pressure by either dilating (to lower blood pressure) or constricting (to raise blood pressure).

The importance of blood pressure in the kidneys is amplified because of the central electrical, mechanical, and hormonal role of the kidneys play. For example, the kidneys affect blood pressure by signaling the need for increased or lowered pressure through the SNS (electrical), by filtering blood and controlling the amount of fluid in the body (mechanical), and by releasing key hormones that influence the activities of the heart and blood vessels to maintain cardiovascular homeostasis (hormonal). The kidneys send and receive electrical signals from the SNS and thereby affect the other organs related to blood pressure control. They receive SNS signals primarily from the brain, which partially control the mechanical and hormonal functions of the kidneys. At the same time, the kidneys also send signals to the rest of the SNS, which may boost the level of sympathetic activation of all the other organs in the system, effectively amplifying electrical signals in the system and the corresponding blood pressure effects. From the mechanical perspective, the kidneys are responsible for controlling the amount of water and sodium in the blood, directly affecting the amount of fluid within the circulatory system. If the kidneys allow the body to retain too much fluid, the added fluid volume raises blood pressure. Lastly, the kidneys produce blood pressure regulating hormones including renin, an enzyme that activates a cascade of events through the renin-angiotensin-aldosterone system (RAAS). This cascade, which includes vasoconstriction, elevated heart rate, and fluid retention, may be triggered by sympathetic stimulation. The RAAS operates normally in non-hypertensive patients but may become overactive among hypertensive patients. The kidney also produces cytokines and other neurohormones in response to elevated sympathetic activation that may be toxic to other tissues, particularly the blood vessels, heart, and kidney. As such, overactive sympathetic stimulation of the kidneys may be responsible for much of the organ damage caused by chronic high blood pressure.

Thus, overactive sympathetic stimulation of the kidneys plays a significant role in the progression of hypertension, CHF, CRF, and other cardio-renal diseases. Heart failure and hypertensive conditions often result in abnormally high sympathetic activation of the kidneys, creating a vicious cycle of cardiovascular injury. An increase in renal sympathetic nerve activity leads to the decreased removal of water and sodium from the body, as well as increased secretion of renin, which leads to vasoconstriction of blood vessels supplying the kidneys. Vasoconstriction of the renal vasculature causes decreased renal blood flow, which causes the kidneys to send afferent SNS signals to the brain, triggering peripheral vasoconstriction and increasing a patient's hypertension. Reduction of sympathetic renal nerve activity, e.g., via renal neuromodulation or denervation of the renal nerve plexus, may reverse these processes.

Efforts to control the consequences of renal sympathetic activity have included the administration of medications such as centrally acting sympatholytic drugs, angiotensin converting enzyme inhibitors and receptor blockers (intended to block the RAAS), diuretics (intended to counter the renal sympathetic mediated retention of sodium and water), and beta-blockers (intended to reduce renin release). The current pharmacological strategies have significant limitations, including limited efficacy, compliance issues, and side effects.

As an example, thermal neuromodulation by either intravascular heating or cooling may decrease renal sympathetic activity by disabling the efferent and/or afferent sympathetic nerve fibers that surround the renal arteries and innervate the kidneys through renal denervation, which involves selectively disabling renal nerves within the sympathetic nervous system (SNS) to create at least a partial conduction block within the SNS.

Also, several forms of renal injury or stress may induce activation of the renal afferent signals (*e.g.*, from the kidney to the brain or the other kidney). For example, renal ischemia, a reduction in stroke volume or renal blood flow, may trigger activation of renal afferent nerve activity. Increased renal afferent nerve activity results in increased systemic sympathetic activation and peripheral vasoconstriction (narrowing) of blood vessels. Increased vasoconstriction results in increased resistance of blood vessels, which results in hypertension. Increased renal efferent nerve activity (*e.g.*, from the brain to the kidney) results in further increased afferent renal nerve activity and activation of the RAAS cascade, inducing increased secretion of renin, sodium retention, fluid retention, and reduced renal blood flow through vasoconstriction. The RAAS cascade also contributes to systemic vasoconstriction of blood vessel, thereby exacerbating hypertension. In addition, hypertension often leads to vasoconstriction and atherosclerotic narrowing of blood vessels supplying the kidneys, which causes renal hypoperfusion and triggers increased renal afferent nerve activity. In combination this cycle of factors results in fluid retention and increased workload on the heart, thus contributing to the further cardiovascular and cardio-renal deterioration of the patient.

Furthermore, because renal denervation reduces overactive SNS activity, it may be valuable in the treatment of several other medical conditions related to hypertension. These conditions, which are characterized by increased SNS activity, include left ventricular hypertrophy, chronic renal disease, chronic heart failure, insulin resistance (diabetes and metabolic syndrome), cardio-renal syndrome, osteoporosis, and sudden cardiac death. For example, other benefits of renal denervation may theoretically include: reduction of insulin resistance, reduction of central sleep apnea, improvements in perfusion to exercising muscle in heart failure, reduction of left ventricular hypertrophy, reduction of ventricular rates in patients with atrial fibrillation, abrogation of lethal arrhythmias, and slowing of the deterioration of renal function in chronic kidney disease. Moreover, chronic elevation of renal sympathetic tone in various disease states that exist with or without hypertension may play a role in the development of overt renal failure and end-stage renal disease. Because the reduction of afferent renal sympathetic signals contributes to the reduction of systemic sympathetic stimulation, renal denervation may also benefit other organs innervated by sympathetic nerves. Thus, renal denervation may also alleviate various medical conditions, even those not directly associated with hypertension.

As noted, renal denervation is a treatment option for resistant hypertension. However, the efficacy of renal denervation may be very variable between patients. Recent studies indicate that the velocity of the pressure/flow pulse (pulse wave velocity or PWV) inside the main renal artery may be indicative of the outcome of renal denervation. Additionally, the PWV in patients with resistant hypertension may be very high (*e.g.,* more than 20 m/s), which may make it difficult to determine the PWV in the relatively short renal arteries (*e.g.*, 5-8 cm in length).

While existing diagnostic and/or treatment methods have been generally adequate for their intended purposes, they have not been entirely satisfactory in all respects. The devices, systems, and associated methods of the present disclosure overcome one or more of the shortcomings of the prior art.

WO 00/55579 A2 discloses a method and devices for determining volume flow, blood velocity profile, artery wall properties of a tubular conduit system and stenosis identification and localization by introducing an artificial pressure or flow excitation signal (a single signal or multiple signals) into the blood vessel (or in any other tubular flowing fluid conduits), and or using natural heart beat signals measurement and analysis of the vessel wall displacements or vessel diameter changes.

US 2009/0270695 A1 discloses an apparatus and methods for continuous intravascular measurement of whole blood concentration, blood pressure, and pulse pressure. The intravascular catheter incorporates a sensor to measure whole blood sound velocity, attenuation, backscatter amplitude, and blood flow velocity and also incorporates existing technologies for multiple physiologic measurements of whole blood. Pulse wave velocity and wave intensity are derived mathematically for purposes of estimating degree of local vascular tone.

## SUMMARY OF THE INVENTION

The present disclosure describes calculation of a physiological quantity known as a pulse wave velocity (PWV). The PWV represents the velocity of the pressure and flow waves that propagate through blood vessels of a patient as a result of the heart pumping. Recent studies indicate that the PWV within the renal artery, which is an artery that supplies blood to the kidney, is indicative of whether a therapeutic procedure known as renal denervation will be successful in the patient. Renal denervation is used to treat hypertension. As described in more detail herein, PWV can be calculated using measurements of velocity and pressure within the vessel. The velocity data can be obtained by an ultrasound imaging device positioned outside of the body. The pressure data can be obtained by an intravascular pressure-sensing device that is positioned within the vessel. By obtaining the velocity data and the pressure data simultaneously and from the same area within the vessel, a mathematical relationship between velocity and pressure is used to calculate the PWV. The calculated PWV for the patient can then be used to determine whether the patient is good candidate for treatment. For example, the PWV measurement result can be used to perform patient stratification for the renal denervation, before performing the treatment, by predicting the efficacy of renal denervation based on PWV.

In one embodiment, an apparatus for pulse wave velocity (PWV) determination in a vessel is provided. The apparatus includes an intravascular device sized and shaped for positioning within the vessel, the intravascular device including a flexible elongate member having a proximal portion and a distal portion; and a pressure sensor coupled to the distal portion of the flexible elongate member, the pressure sensor configured to monitor a pressure of a fluid within the vessel; an imaging device configured to monitor a velocity of the fluid within the vessel, the imaging device being configured to obtain imaging data from an external ultrasound transducer configured for positioning proximate to a body portion including the vessel; and a processing system in communication with the intravascular device and the imaging device, the processing system configured to: synchronize the monitoring of the pressure within the vessel by the pressure sensor with the monitoring of the velocity within the vessel by the imaging device; and determine the pulse wave velocity of the fluid within the vessel based on the pressure and the velocity within the vessel.

In one embodiment, a method of determining pulse wave velocity (PWV) in a vessel is provided. The method includes monitoring pressure associated with a fluid within the vessel, the monitoring pressure being performed using an intravascular device including a pressure sensor and positioned within the vessel; monitoring velocity associated with the fluid within the vessel, the monitoring velocity being performed using an imaging device positioned outside of a body portion including the vessel; receiving pressure data associated with the monitoring of the pressure; receiving velocity data associated with the monitoring of the velocity; and determining the pulse wave velocity of the fluid within the vessel based on the pressure data and the velocity data wherein the monitoring of the pressure within the vessel by the pressure sensor is synchronized by a processing system with the monitoring of the velocity within the vessel by the imaging device.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

The invention is defined by appended claims 1-13.

## BRIEF DESCRIPTION OF THE DRAWINGS

The accompanying drawings illustrate embodiments of the devices and methods disclosed herein and together with the description, serve to explain the principles of the present disclosure.

Fig. 1A is a diagrammatic schematic view of an exemplary intravascular system.
Fig. 1B is a diagrammatic schematic view of an exemplary intravascular system.
Fig. 1C is a diagrammatic schematic view of an exemplary intravascular system.
Fig. 2 is a schematic diagram illustrating an intravascular device positioned within renal anatomy.
Fig. 3A is a diagrammatic illustration of obtaining pressure data and velocity data within a vessel using an exemplary intravascular system.
Fig. 3B is a diagrammatic illustration of obtaining pressure data and velocity data within a vessel using an exemplary intravascular system.
Fig. 4 is an illustration of an exemplary screen display of an intravascular system.
Fig. 5 is a flowchart illustrating a method of calculating a pulse wave velocity.

## DETAILED DESCRIPTION OF THE EMBODIMENTS

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

The present disclosure relates generally to devices, systems, and methods for determining and measuring pulse wave velocity (PWV) in a main renal artery. An apparatus for PWV determination includes an intravascular device having a pressure sensor. The intravascular device is sized and shaped for positioning within a vessel, such as the main renal artery. While positioned within the vessel, the intravascular device obtains pressure measurements of the fluid, such as blood, flowing within the vessel. The apparatus also includes an imaging device, such as an external ultrasound device. The imaging device can be positioned outside of the body, such as in contact with and/or adjacent to a body portion including the vessel. The imaging device can obtain velocity data, including a magnitude and direction, associated with the fluid flow within the vessel. The apparatus may further include a computing device, such as a controller or computing device, in communication with intravascular device and the imaging device. The computing device calculates the PWV based on the obtained pressure data and velocity data.

The PWV may be predictive of the outcome of renal denervation in treating resistive hypertension. As described herein, the computing device can output the calculated PWV to a display. A clinician may make therapeutic and/or diagnostic decisions, taking the PWV into consideration, such as whether to recommend the patient for a renal denervation procedure. In some instances, the computer system can determine and output a therapy recommendation or a likelihood-of-success prediction to the display, based on the PWV and/or other patient data. That is, the computer system may utilize the PWV to identify which patients are more likely and/or less likely to benefit from renal denervation.

Fig. 1A is a diagrammatic schematic view of an exemplary intravascular system 100 according to some embodiments of the present disclosure. The system 100 may include an intravascular device 110, an imaging device 190, a processing system 130, and a display 160. The system 100 may also include an interface module 120, a pressure console 112, an ultrasound console 192, and an electrocardiograph (ECG) console 102. The system 100 may be configured to perform pulse wave velocity (PWV) determination in a vessel 80 within a body portion. The intravascular system 100 may be referred to as a stratification system in that the PWV may be used for patient stratification for treatment purposes. For example, the PWV determination in the renal arteries may be utilized to determine whether a patient is suitable for and/or likely to benefit from renal artery denervation. Based on the PWV determination, the intravascular system 100 may be used to classify one or more patients into groups respectively associated with varying degrees of predicted therapeutic benefit of renal denervation. Any suitable number of groups or categories are contemplated. For example, the groups may include groups respectively for those patients with low, moderate, and/or high likelihood of therapeutic benefit from renal denervation, based on the PWV. Based on the stratification or classification, the system 100 can recommend the degree to which one or more patients are suitable candidates for renal denervation.

The vessel 80 may represent fluid-filled or surrounded structures, both natural and man-made. The vessel 80 may be within a body of a patient. The vessel 80 may be a blood vessel, as an artery or a vein of a patient's vascular system, including cardiac vasculature, peripheral vasculature, neural vasculature, renal vasculature, and/or or any other suitable lumen inside the body. For example, the device 110 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the heart, chambers or other parts of the heart, and/or other systems of the body. In addition to natural structures, the device 110 may be may be used to examine man-made structures such as, but without limitation, heart valves, stents, shunts, filters and other devices. Walls of the vessel 80 define a lumen 82 through which fluid flows within the vessel 80.

The vessel 80 may be located within the body portion 180. When the vessel 80 is the renal artery, the patient body portion 180 may include the abdomen, lumbar region, and/or thoracic region. Generally, vessel 80 may be located within any portion 180 of the patient body, including the head, neck, chest, abdomen, arms, groin, legs, etc.

The intravascular device 110 may be a catheter, guide wire, or guide catheter, and/or other long, thin, long, flexible structure that is sized and shaped be inserted into the lumen 82 of the vessel 80. In that regard, the intravascular device 110 includes a flexible elongate member 170 having a distal portion 172 and a proximal portion 174. The illustrated embodiments of the intravascular device 110 may have a cylindrical profile with a circular cross-sectional profile, which defines an outer diameter of the intravascular device 110. In other instances, all or a portion of the intravascular device may have other geometric cross-sectional profiles (*e.g.,* oval, rectangular, square, elliptical, *etc.*) or non-geometric cross-sectional profiles. In some embodiments, the intravascular device 110 may or may not include a lumen extending along all or a portion of its length for receiving and/or guiding other instruments. If the intravascular device 110 includes a lumen, the lumen may be centered or offset with respect to the cross-sectional profile of the device 110.

The intravascular device 110, or the various components thereof, may be manufactured from a variety of materials, including, by way of non-limiting example, plastics, polytetrafluoroethylene (PTFE), polyether block amide (PEBAX), thermoplastic, polyimide, silicone, elastomer, metals, such as stainless steel, titanium, shape-memory alloys such as Nitinol, and/or other biologically compatible materials. In addition, the intravascular device may be manufactured in a variety of lengths, diameters, dimensions, and shapes, including a catheter, guide wire, a combination of catheter and guide wire, *etc.* For example, in some embodiments the flexible elongate member 170 may be manufactured to have length ranging from approximately 115 cm - 155 cm. In one particular embodiment, the flexible elongate member 170 may be manufactured to have length of approximately 135 cm. In some embodiments, the flexible elongate member 170 may be manufactured to have an outer transverse dimension or diameter ranging from about 0.35 mm - 2.67 mm (1 Fr - 8 Fr). In one embodiment, the flexible elongate member 170 may be manufactured to have a transverse dimension of 2 mm (6 Fr) or less, thereby permitting the intravascular device 110 to be configured for insertion into the renal vasculature of a patient. These examples are provided for illustrative purposes only, and are not intended to be limiting. Generally, the intravascular device 110 is sized and shaped such that it may be moved inside the vasculature (or other internal lumen(s)) of a patient such that the pressure of a vessel 80 may be monitored from within the vessel 80.

In the embodiment of Fig. 1A, the intravascular device 110 includes a single sensor 204 disposed at a distal portion 172 of the flexible elongate member 170. In other embodiments, such as an intravascular system 200 of Fig. 1B, the intravascular device 110 includes two sensors 202, 204 disposed at the distal portion 172 of the flexible elongate member 170. The sensors 202, 204 may be disposed a known distance D1 apart from one another along the length of the flexible elongate member 170. In some embodiments, the distance D1 is fixed distance between approximately 0.5 and approximately 10 cm. In some embodiments, the distance D1 is between approximately 0.5 cm and approximately 2 cm. In some embodiments, blood pressure measurements may be used to identify pulse waves passing through the vessel.

The sensors 202, 204 may be configured to collect data about conditions within the vessel 80, and in particular, monitor a pressure within the vessel 80. Furthermore, the sensors 202, 204 may periodically measure the pressure of fluid (*e.g.,* blood) at the location of the sensors 202, 204 inside the vessel 80. In an example, the sensors 202, 204 are capacitive pressure sensors, or in particular, capacitive MEMS pressure sensors. In another example, the sensors 202, 204 are piezo-resistive pressure sensors. In yet another example, the sensors 202, 204 are optical pressure sensors. In some instances, the sensors 202, 204 include components similar or identical to those found in commercially available pressure monitoring elements such as the PrimeWire PRESTIGE® pressure guide wire, the PrimeWire® pressure guide wire, and the ComboWire® XT pressure and flow guide wire, each available from Volcano Corporation. The intravascular device 110 is in communication with the interface module 120, the pressure console 112, and/or the processing system 130 such that the intravascular data obtained by the sensors 202, 204 is transmitted by the intravascular device 110 and received at the processing system 130.

In one embodiment, the sensors 202, 204 are disposed circumferentially around a distal portion of the intravascular device 110. In another embodiment, the sensors 202, 204 are contained within the body of the intravascular device 110. In other embodiments, the sensors 202, 204 are disposed longitudinally and/or radially across the intravascular device 110 in a manner that permits the fluid within the vessel 80 to exert pressure on the sensors 202, 204. The sensors 202, 204 may include one or more transducer elements. The sensor 202 and/or the sensor 204 may be movable along a length of the intravascular device 110 and/or fixed in a stationary position along the length of the intravascular device 110. The sensors 202, 204 may be part of a planar or otherwise suitably-shaped array of sensors of the intravascular device 110. In some embodiments, the outer diameter of the flexible elongate member 170 is equal to or larger than the outer diameter of the sensors 202, 204. In some embodiments, the outer diameter of the flexible elongate member 170 and sensors 202, 204 are equal to or less than about 1 mm, which may help to minimize the effect of the intravascular device 110 on pressure measurements within the vessel 80. In particular, since a renal artery generally has a diameter of approximately 5 mm, a 1 mm outer diameter of the intravascular device 110 may obstruct less than 4% of the vessel.

In some embodiments, one or both of the sensors 202, 204 may not be part of the same intravascular device 110. For example, as shown the intravascular system 250 of Fig. 1C, the sensors 202, 204 may be coupled to separate intravascular devices 110, 113. In that regard, the intravascular device 113 includes a flexible elongate member 171. The sensor 202 is coupled to a distal portion of the flexible elongate member 171. The sensor 204 may be coupled to a distal portion of the flexible elongate member 170 of the intravascular device 110. In some embodiments, the intravascular device 113 may be a catheter and the intravascular device 110 may be a guide wire extending through a lumen of the catheter such that the sensor 204 is positioned distally of the sensor 202 within the vessel 80. In such an arrangement, sensor 202 may be configured to obtain a proximal pressure measurement and the sensor 204 may be configured to obtain a distal pressure measurement. The intravascular device 113 may be in communication with the processing system 130 via the pressure console 112 and/or the interface module 123.

Referring to Figs. 1A, 1B, and 1C, the imaging device 190 is configured to obtain imaging data associated with the vessel 80. The imaging device 190 may be an ultrasound imaging device in some embodiments. In that regard, the imaging device 190 can include a probe 194 having an ultrasound transducer 196. The transducer 196 may be a single transducer element, an array of transducer elements, and/or other suitable arrangement of transducer elements. In some instances, the probe 194 is sized and shaped for handheld grasping by a user, such as the clinician. In some embodiments, such as those illustrated in Figs. 3A and 3B, the probe 194 is coupled to and/or disposed within a holder 210. As described herein, the holder 210 is configured to move the probe 194 into a position and/or orientation for the transducer 196 to obtain imaging data in a desired manner from the vessel 80.

Generally, as shown in Figs. 3A and 3B, the transducer 196 is configured to emit ultrasonic energy 198 in order to create an image of the vessel 80 and/or surrounding anatomy within the body portion 180. Ultrasonic waves 198 are partially reflected by discontinuities arising from tissue structures (such as the various layers of the vessel wall), red blood cells, and other features of interest. Echoes from the reflected waves are received by the transducer and passed along to the ultrasound console 192 and/or the processing system 130. The ultrasound console 192 and/or the processing system 130 processes the received ultrasound echoes to produce an image of the vessel 80 and/or the body portion 180 based on the acoustic impedance associated with the ultrasound echoes. The image may be a two-dimensional cross-sectional image or a three-dimensional image of the vessel. The imaging device 190 and/or the ultrasound console 192 may include features similar or identical to those found in commercially available ultrasound imaging elements such as the EPIQ, Affiniti, and/or CX50 ultrasound systems, each available from Koninklijke Philips N.V.

The probe 194 and/or the transducer 196 are positioned outside of the body portion 180. However, the probe 194 and/or the transducer 196 may be positioned proximate to and/or in contact with the body portion 180 including the vessel 80. The clinician and/or the holder 210 may contact the transducer 196 to the body portion 180 such that the anatomy is compressed in a resilient manner. The view of the anatomy shown in the ultrasound image depends on the position and orientation of the probe 194. To obtain imaging data of the vessel 80, including the sensors 202, 204, the probe 194 can be suitably positioned either manually by a clinician and/or automatically by the holder 210 so that the transducers 196 emits ultrasound waves and receives ultrasound echoes from the appropriate portion of the vessel 80.

Imaging data obtained by the imaging device 190 can include velocity data and/or lumen data. In this regard, the vessel 80 includes anatomical structure, such as vessel walls that define the lumen 82, as well as fluid within the 80. Fluid within the vessel 80 is moving relative to the imaging device 190. Lumen data obtained by the imaging device 190 may be representative of relatively stationary anatomy, including the vessel walls. The ultrasound console 192 and/or the processing system 130 generate a B-mode (brightness mode) imaging of anatomy corresponding to the varying acoustic impedance of the received ultrasound echoes. The lumen data can be used to evaluate the diameter, cross-sectional area, volume, and/or other geometric data associated with the vessel 80. The velocity data obtained by the imaging device 190 is representative of the magnitude and/or the direction of fluid flow within the vessel 80. In that regard, the velocity data may be indicative of localized magnitude/direction of fluid flow (e.g., for each pixel in the image of the vessel 80). Various techniques exist for determining velocity data, including Doppler flow and vector flow. In Doppler flow imaging, the frequency shift associated with ultrasound energy obtained by the transducer 196 is calculated and used to determine the speed and direction of the fluid flow. Doppler flow imaging may depend on angle of the transducer 196 relative to the fluid flow and be limited to one-dimensional flow information. Vector flow imaging derives three-dimensional flow information from the ultrasound data obtained by the transducer 196 so that angle-independent visualization of fluid flow is provided.

The lumen data and the velocity data are often combined for display on the display 160, as shown for example in Fig. 4. Doppler flow data 302 may be illustrated as different colors indicative of varying magnitudes and direction of fluid flow, as provided in the key or legend 306. Vector flow data may be illustrated symbolically such as with arrows 304 having varying magnitudes and directions. The Doppler flow and/or vector flow data may be referenced as a velocity map that is overlaid on the B-mode image of the vessel 80 illustrating the vessel geometry.

Referring again to Figs. 1A, 1B, and 1C, the systems 100, 200, and 250 can include an electrocardiograph (ECG) console configured to obtain ECG data from electrodes positioned on the patient. ECG signals are representative of electrical activity of the heart and can be used to identify the patient's cardiac cycle and/or portions thereof. As described in greater detail herein, the processing system 130 can utilize different formula to calculate PWV based on whether the velocity data obtained by the imaging device 190 and/or the pressure data obtained by the intravascular device 110 is obtained over an entire cardiac cycle and/or a portion thereof. The ECG data can be used to identify the beginning and ending of the previous, current, and next cardiac cycle(s), the beginning and ending of systole, the beginning and ending of diastole, among other portions of the cardiac cycle. Generally, one or more identifiable feature of the ECG signal (including without limitation, the start of a P-wave, the peak of a P-wave, the end of a P-wave, a PR interval, a PR segment, the beginning of a QRS complex, the start of an R-wave, the peak of an R-wave, the end of an R-wave, the end of a QRS complex (J-point), an ST segment, the start of a T-wave, the peak of a T-wave, and the end of a T-wave) can be utilized to select relevant portions of the cardiac cycle. The ECG console 102 may include features similar or identical to those found in commercially available ECG elements such as the PageWriter cardiograph system available from Koninklijke Philips N.V.

In other embodiments, the pressure data obtained by the intravascular device 110 is used to identify relevant portions of the cardiac cycle. In that regard, the pressure data may be used in lieu of or in addition to the ECG data. For example, the minimum value, the maximum value, slope, and/or other values of a pressure waveform representative of the obtained pressure data may be indicative of cardiac cycle(s) and/or portions thereof, including systole and diastole.

The processing system 130 may be in communication with the intravascular device 110. For example, the processing system 130 may communicate with the intravascular device 110, including the sensor 202 and/or the sensor 204, through an interface module 120 and/or pressure console 112. The processor 140 may send commands and receive responses from the intravascular device 110. In some implementations, the processor 140 controls the monitoring of the pressure within the vessel 80 by the sensors 202, 204. In particular, the processor 140 may be configured to trigger the activation of the sensors 202, 204 to measure pressure at specific times. Data from the sensors 202, 204 may be received by the processing engine 140 of the processing system 130. In other embodiments, the processing engine 140 is physically separated from the intravascular device 110 but in communication with the intravascular device 110 (*e.g.,* via wireless communications). In some embodiments, the processing engine 140 is configured to control the sensors 202, 204. Similarly, in embodiments including two intravascular devices (Fig. 1C), the processing system 130 may communicate with the intravascular device 113 through the interface module 123 and/or pressure console 112.

Similarly, the processing system 130 may be in communication with the imaging device 190. For example, the processing system 130 may communicate with the imaging device 190, including the transducer 196. The processor 140 may send commands and receive responses from the imaging device 190. In some implementations, the processor 140 controls the monitoring of the velocity and/or vessel geometry within the vessel 80 by the transducer 196. In particular, the processor 140 may be configured to trigger the activation of the transducer 196 to obtain velocity data and/or lumen data at specific times. Data from the transducer 196 may be received by a processing engine 140 of the processing system 130. In other embodiments, the processing engine 140 is physically separated from the intravascular device 110 but in communication with the imaging device 190 (*e.g.,* via wireless communications). In some embodiments, the processing engine 140 is configured to control the transducer 196.

The processor 140 may include an integrated circuit with power, input, and output pins capable of performing logic functions such as commanding the sensors and receiving and processing data. The processor 140 may include any one or more of a microprocessor, a controller, a digital signal processor (DSP), an application specific integrated circuit (ASIC), a field-programmable gate array (FPGA), or equivalent discrete or integrated logic circuitry. In some examples, processor 140 may include multiple components, such as any combination of one or more microprocessors, one or more controllers, one or more DSPs, one or more ASICs, or one or more FPGAs, as well as other discrete or integrated logic circuitry. The functions attributed to processor 140 herein may be embodied as software, firmware, hardware or any combination thereof.

The processing system 130 may include one or more processors 140 or programmable processor units running programmable code instructions for implementing the pulse wave velocity determination methods described herein, among other functions. The processing system 130 may be integrated within a computer and/or other types of processor-based devices. For example, the processing system 130 may be part of a console, tablet, laptop, handheld device, or other controller used to generate control signals to control or direct the operation of the intravascular device 110. In some embodiments, a user may program or direct the operation of the intravascular device 110 and/or the imaging device 190, and/or control aspects of the display 160. In some embodiments, the processing system 130 may be in direct communication with the intravascular device 110 (e.g., without an interface module 120) and/or the imaging device 190, including via wired and/or wireless communication techniques.

The processing system 130 may be in communication with the intravascular device 110 via the interface module 120 and/or the pressure console 112. The interface module 120 can include circuitry configured to facilitate transmission of control signals from the processing system 130 to the intravascular device 110, as well as the transmission of pressure data from the intravascular device 110 to the processing system 130. In some embodiments, the interface module 120 can provide power to the sensors 202, 204. In some embodiments, the interface module can perform signal conditioning and/or pre-processing of the pressure data prior to transmission to the processing system 130.

The pressure console 112 may be representative of processing system associated specifically with the intravascular device 110. In that regard, the pressure console 112 may include a processing engine and processing memory similar to the processing engine 140 and the processing memory 150 of the processing system 130. In some instances, the pressure console 112 processes the pressure data obtained by the sensors 202, 204 to measure pressure associate with the fluid flow within the vessel 80. The processed data may be transmitted by the pressure console 112 to the processing system 130 for further processing in combination with the imaging data obtained by the imaging device 190, including calculation of the PWV based on the pressure data and the imaging data. In some instances, the pressure console 112 and/or the interface module 120 may be omitted, and the intravascular device 110 may be directly in communication with the processing system 130.

The processing system 130 may be in communication with the imaging device 190 via the ultrasound console 192. As similarly described with respect to the pressure console 112, the ultrasound console 192 may be representative of processing system associated specifically with the imaging device 190. In that regard, the ultrasound console 192 may include a processing engine and processing memory similar to the processing engine 140 and the processing memory 150 of the processing system 130. In some instances, the ultrasound console 192 processes the imaging data, including the velocity data and/or lumen data, obtained by the transducer 196 to generate images representative of the vessel geometry and/or fluid flow velocity within the vessel 80. The processed data may be transmitted by the ultrasound console 192 to the processing system 130 for further processing in combination with the pressure data obtained by the intravascular device 110, including calculation of the PWV based on the pressure data and the imaging data. In some instances, the ultrasound console 192 may be omitted, and the imaging device 190 may be directly in communication with the processing system 130.

Moreover, in some embodiments, the pressure console 112, the interface module 120, the ultrasound console 192, and/or the processing system 130 are collocated and/or part of the same system, unit, chassis, or module. Together the pressure console 112, the interface module 120, the ultrasound console 192, and/or the processing system 130 assemble, process, and render the imaging data obtained by the imaging device 190 and the pressure data obtained by the intravascular device 110 for display as an image on a display 160. For example, in various embodiments, the interface module 120 and/or processing system 130 generate control signals to configure the sensors 202, 204, generate signals to activate the sensors 202, 204, perform calculations of pressure data, perform amplification, filtering, and/or aggregating of pressure data, and format the pressure data as an image for display. Similarly, in various embodiments, the processing system 130 generates control signals to configure the transducer 196, generate signals to activate the transducer 196, perform calculations of imaging data, perform amplification, filtering, and/or aggregating of imaging data, and format the imaging data as an image for display. The allocation of these tasks and others may be distributed in various ways between the pressure console 112, the interface module 120, the ultrasound console 192, and/or the processing system 130.

The processing system 130 is configured to synchronize the collection of the pressure data by the intravascular device 110 and the ultrasound data by the imaging device 190. In some instances, the interface module 120, the pressure console 112, and/or ultrasound console 192, as well as the processing system 130 can include a timer. By communicating to the interface module 120, the pressure console 112, and/or ultrasound console 192, the processing system 130 can synchronize the timer of the interface module 120, the pressure console 112, and/or ultrasound console 192 with the processor timer. Additionally, the interface module 120, the pressure console 112, and/or ultrasound console 192 can do the sampling of the signals received from sensors 202, 204 and the imaging device 190 and can include a time stamp to the sampled data and then send the time-stamped sampled data to the processing system 130 such that the pressure data associated with the monitoring of the pressure within the vessel and the velocity data associated monitoring the velocity within the vessel, received by processing system 130 , is time-stamped and processing system 130 can synchronize the data based on the received time stamps.

Alternatively, instead of the interface module 120, the pressure console 112, and/or ultrasound console 192, the sensors 202, 204, and the imaging device 190 can perform the sampling and send the sampled data to the processing system 130. The intravascular device 110 can include one or more timers for the sensors 202, 204, and the imaging device 190 can include a timer. The processing system 130, by communicating to intravascular device 110 and the imaging device 190, can synchronize data collection by the sensors 202, 204 and the transducer 196 with the processor timer. Thus, the data obtained by the imaging device 190 and the sensors 202, 204 can include a time stamp. The interface module 120, the pressure console 112, and/or ultrasound console 192 can use the time stamps to synchronize the obtained data and then send the data to the processing system 130. In another example, the interface module 120, the pressure console 112, and/or ultrasound console 192 can send the time-stamped data obtained by sensors 202, 204 and the imaging device 190 to the processing system 130. The processing system 130 can synchronize the data based on the received time stamps.

In some instances, ECG data obtained by the ECG console 102 can be used to synchronize collection of the pressure data by the sensors 202, 204 and the imaging data by the imaging device 190. For example, the processing system 130 can provide controls signals to the sensors 202, 204 and the imaging device 190 to simultaneously sample data at the same time in the cardiac cycle. One or more features of the ECG data may be used to determine the point during the cardiac cycle at which to simultaneously sample data.

In some instances, the sensors 202, 204 and the imaging device 190 obtain data at different rates. Accordingly, the processing system 130 is configured to align acquisition of pressure data and imaging data by offsetting the control signal transmission to sample data. For example, if the sensors 202, 204 sample data at a higher frequency than the imaging device 190, then the processing system 130 can transmit the control signal to the imaging device 190 before transmitting the control signal to the sensors 202, 204. In this manner, because the imaging device 190 has relatively more time to obtain the imaging data than the sensors 202, 204 have time to obtain pressure data, the imaging data and the pressure data can be obtained at the same time. The processing system 130 may implement a calibration procedure to determine any necessary offset in the activation signals to the imaging device 190 and the sensors 202, 204.

In some embodiments, the processing system 130 is configured to determine PWV based on the pressure data obtained by the sensors and the velocity/imaging data obtained by the imaging device 190. Generally, the PWV refers to the propagation of the pressure/flow waves within the vessel 80 that is determined by its distensibility. As described herein, the velocity data obtained by the imaging device 190 is a localized, instantaneous measurement of the velocity of blood within the vessel. Accordingly, the velocity data obtained by the imaging device 190 together with the pressure data obtained by intravascular device 110 is used to calculate the PWV. This method of determining PWV is particularly advantageous for relatively short vessels, such as the renal artery.

As described herein, the pressure data and the velocity data can be both simultaneously obtained and obtained in the same area of the vessel 80. The processing system 130 can utilize various mathematical relationships between the pressure and velocity within the vessel, as well as other factors, to compute the PWV. One method to determine the PWV is by utilizing the "water hammer" equation during a reflection free period of the cardiac cycle (*e.g.,* early systole):

$$PWV = \frac{1}{\rho}\frac{dP}{dU}, \qquad (1)$$

where $dP$ is a change in the pressure over a time interval, $dU$ is a change in the velocity over the time interval, and $\rho$ is a density of the fluid within the vessel. Alternatively, the following relation may be used to calculate the PWV by summation over the whole cardiac cycle:

$$PWV = \frac{1}{\rho}\sqrt{\frac{\sum dP^2}{\sum dU^2}} \qquad (2)$$

where *dP* is a change in the pressure over a time interval, *dU* is a change in the velocity over the time interval, and $\rho$ is a density of the fluid within the vessel.

The processing system 130 can select an appropriate mathematical relationship based on whether the pressure data and/or the velocity data are obtained over an entire cardiac cycle or a portion thereof. In that regard, the pressure data and/or the velocity data may be gated based on the ECG data. Thus, the PWV may be calculated using equation (1) above with only the portion of pressure data and velocity data obtained during early systole, determined using the identifiable features of the ECG signal. Similarly, the relevant portion of the pressure data and the velocity data during one or more cardiac cycle(s) can be identified using the ECG signal and used to calculate the PWV with equation (2). In some instances, the features of the pressure waveform (e.g., maximum, minimum, slope, etc.) may be utilized in addition to or in lieu of the ECG data to gate the pressure data and the velocity data. In some instances, the user provides a user input to select the desired mathematical relationship to calculate the PWV, and the processing system 130 can direct collection of the pressure data and the velocity data accordingly.

The processing system 130 is additionally configured to monitor motion of intravascular device 110, the sensors 202, 204, and/or the imaging device 190. In that regard, the sensors 202, 204 and/or the imaging device 190 may move beyond a threshold amount, which renders the data collected during the movement unreliable for PWV calculation. For example, the patient may unexpectedly move, or the clinician may unintentionally move the intravascular device 110 and/or the imaging device 190. In such instances, the processing system 130 withholds determination of the PWV. In this manner, only reliable PWV calculations are displayed for the clinician and/or utilized by the processing system 130 to make therapy recommendations.

Various peripheral devices may enable or improve input and output functionality of the processing system 130. Such peripheral devices may include, but are not necessarily limited to, standard input devices (such as a mouse, joystick, keyboard, *etc.*), standard output devices (such as a printer, speakers, a projector, graphical display screens, *etc.*), a CD-ROM drive, a flash drive, a network connection, and electrical connections between the processing system 130 and other components of the system 100, system 200 and/or system 250. By way of non-limiting example, the processing system 130 may manipulate signals from the intravascular device 110 and/or the imaging device 190 to generate an image on the display 160 representative of the acquired pressure data, imaging data, PWV calculations, and/or combinations thereof. In some instances, the display 160 may be a touch-screen display. Such peripheral devices may also be used for downloading software containing processor instructions to enable general operation of the intravascular device 110, the imaging device 190, and/or the processing system 130, and for downloading software implemented programs to perform operations to control, for example, the operation of any auxiliary devices coupled to the intravascular device 110 and/or the imaging device 190. In some embodiments, the processing system 130 may include a plurality of processing units employed in a wide range of centralized or remotely distributed data processing schemes.

The memory 150 may be a semiconductor memory such as, for example, read-only memory, a random access memory, a FRAM, or a NAND flash memory. The memory 150 may interface with the processor 140 such that the processor 140 may write to and read from the memory 150. For example, the processor 140 may be configured to receive data from the intravascular device 110, the interface module 120, the pressure console 112, the imaging device 190, and/or the ultrasound console 192 and write that data to the memory 150. In this manner, a series of data readings may be stored in the memory 150. The processor 140 may be capable of performing other basic memory functions, such as erasing or overwriting the memory 150, detecting when the memory 150 is full, and other common functions associated with managing semiconductor memory.

Fig. 2 illustrates the intravascular device 110 of Fig. 1 disposed within the human renal anatomy. The human renal anatomy includes kidneys 10 that are supplied with oxygenated blood by right and left renal arteries 81, which branch off an abdominal aorta 90 at the renal ostia 92 to enter the hilum 95 of the kidney 10. The abdominal aorta 90 connects the renal arteries 81 to the heart (not shown). Deoxygenated blood flows from the kidneys 10 to the heart via renal veins 101 and an inferior vena cava 111. Specifically, the flexible elongate member 170 of the intravascular device 110 is shown extending through the abdominal aorta and into the left renal artery 81. In alternate embodiments, intravascular device 110 may be sized and configured to travel through the inferior renal vessels 115 as well. Specifically, the intravascular device 110 is shown extending through the abdominal aorta and into the left renal artery 81. In alternate embodiments, the catheter may be sized and configured to travel through the inferior renal vessels 115 as well. In some examples, the right or left renal artery 81 may be the vessel 80 described with respect to Fig. 1a, Fig. 1b, and Fig. 1c.

Left and right renal plexi or nerves 121 surround the left and right renal arteries 81, respectively. Anatomically, the renal nerve 121 forms one or more plexi within the adventitial tissue surrounding the renal artery 81. For the purpose of this disclosure, the renal nerve is defined as any individual nerve or plexus of nerves and ganglia that conducts a nerve signal to and/or from the kidney 10 and is anatomically located on the surface of the renal artery 81, parts of the abdominal aorta 90 where the renal artery 81 branches off the aorta 90, and/or on inferior branches of the renal artery 81. Nerve fibers contributing to the plexi 121 arise from the celiac ganglion, the lowest splanchnic nerve, the corticorenal ganglion, and the aortic plexus. The renal nerves 121 extend in intimate association with the respective renal arteries into the substance of the respective kidneys 10. The nerves are distributed with branches of the renal artery to vessels of the kidney 10, the glomeruli, and the tubules. Each renal nerve 121 generally enters each respective kidney 10 in the area of the hilum 95 of the kidney, but may enter the kidney 10 in any location, including the location where the renal artery 81, or a branch of the renal artery 81, enters the kidney 10.

Proper renal function is essential to maintenance of cardiovascular homeostasis so as to avoid hypertensive conditions. Excretion of sodium is key to maintaining appropriate extracellular fluid volume and blood volume, and ultimately controlling the effects of these volumes on arterial pressure. Under steady-state conditions, arterial pressure rises to that pressure level which results in a balance between urinary output and water and sodium intake. If abnormal kidney function causes excessive renal sodium and water retention, as occurs with sympathetic overstimulation of the kidneys through the renal nerves 121, arterial pressure will increase to a level to maintain sodium output equal to intake. In hypertensive patients, the balance between sodium intake and output is achieved at the expense of an elevated arterial pressure in part as a result of the sympathetic stimulation of the kidneys through the renal nerves 121. Renal denervation may help alleviate the symptoms and sequelae of hypertension by blocking or suppressing the efferent and afferent sympathetic activity of the kidneys 10.

Figs. 3A and 3B illustrate aspects of operation of the intravascular system 200 (Fig. 1B) to obtain imaging data via the imaging device 190 and pressure data via the intravascular device 110. While Figs. 3A and 3B are described using components of the intravascular system 200, it is understood that imaging data and pressure data can be similarly obtained using the components of the intravascular systems 100 and 250 (Figs. 1A and 1C).

Fluid, such as blood, flows within the vessel 80 in the exemplary direction indicated by the arrows 220. The intravascular device 110 is positioned within the vessel such that the fluid surrounds and exerts pressure on the pressure sensors 202, 204. Electrical signals representative of the pressure measured by the sensors 202, 204 are transmitted to the pressure console 112 and/or the processing system 130 for use generating a graphical representation of the pressure data and/or in calculating PWV. The graphical representation of the pressure data can include pressure waveform, numerical values of pressure, etc.

The transducer 196 of the imaging device 190 emits ultrasonic energy 198, which is reflected by vessel 80, the fluid within the vessel 80, and/or other anatomy within the patient body 180. The ultrasound echoes from the reflected ultrasound waves are received by the transducer. Electrical signals representative of the imaging data or ultrasound data are transmitted to the ultrasound console and/or the processing system 130 for use in generating a graphical representation of the ultrasound data and/or in calculating PWV. The graphical representation of the ultrasound data can include a B-mode image and velocity map, numerical values of velocity, etc.

The transducer 196 may be configured to obtain ultrasound data while aligned with the vessel 80. In that regard, the transducer 196 may be aligned with the vessel 80 such that the ultrasound data includes components of the intravascular device 110, including the pressure sensors 202, 204. That is, the B-mode/flow image generated from the ultrasound data includes the portion of the vessel 80 in which the sensors 202, 204 are positioned. In this manner, the velocity data obtained by the imaging device 190 is associated with fluid within the same area of the vessel 80 where the sensors 202, 204 are located. Accordingly, the velocity data and the pressure data are obtained at the same time and from the same location within the vessel.

Figs. 3A and 3B illustrate that the imaging device 190 is positioned within a holder 210 that is configured to move the imaging device 190. The holder 210 may be any suitable mechanical structure that is sized and shaped to accommodate the probe 194. The holder 210 is configured to mechanically position and/or orient the probe 194 relative to the vessel 80 such the desired imaging data, including imaging data within portions the vessel 80 containing the pressure sensors 202, 204, is obtained. The holder 210 may include one or more actuators that, in response to a control signal, translate, rotate, and/or otherwise move the probe 194 with, e.g., six degrees of freedom (three rotational degrees of freedom and three translation degrees of freedom). Three degrees of freedom are illustrated in Figs. 3A and 3B. For example, the imaging device 190 can be translated in directions 212, 214 and rotated in a direction 216. Other translational/rotational degrees of freedom are not shown in Figs. 3A and 3B.

Relative to Fig. 3A, the imaging device 190 is rotated clockwise in the direction 216 in Fig. 3B. The holder 210 may be in communication with the processing system 130, which is configured to transmit control signals to the holder 210 to move the imaging device 190. For example, the processing system 130 transmits a control signal to the holder 210 to move the imaging device 190 to obtain imaging data while aligned with the vessel 80 and/or the sensors 202, 204. In some instances, the processing system 130 utilizes a feedback loop to ensure that the imaging device 190 is properly oriented. For example, the processing system 130 may periodically evaluate the imaging data being obtained by the imaging device 190 to ensure that the vessel 80 and the sensors 202, 204 are properly being interrogated by the transducer 196. The holder 210 moves the imaging device 190 when the vessel 80 and the sensors 202, 204 are not properly within view. The holder 210 may automatically adjust the imaging device 190 into alignment with the vessel 80 and/or the sensors 202, 204 in response to a user input. As shown in the exemplary screen display of Fig. 4, the user can selection the input option 330 to toggle on/off the automatic position/orientation correction provided by the holder 210 and the processing system 130.

Referring again to Figs. 3A and 3B, the sensors 202, 204 can be sensitive to ultrasonic waves such that the sensors are visible in the B-mode/flow image. In the regard, the sensors 202, 204 may be associated with ultrasound echoes with relatively higher amplitude, compared to surrounding regions of fluid within the vessel 80. Thus, the processing system 130 can be configured to determine and track locations of the sensors 202, 204 within the vessel 80 using the imaging data obtained by the imaging device 190. Examples of tracking sensors of an interventional tool, e.g., the intravascular device 110, are described in PCT Patent Application Publication No. WO2011138698A1, which is hereby incorporated by reference in its entirety. Additionally, as shown in Fig. 4, in some instances, the processing system 130 highlights the locations of the sensors 202, 204 in the ultrasound image 300. Indicators 308 are overlaid on the image 300 in positions corresponding to the locations of the sensors 202, 204 in the vessel 80. The user may toggle the sensor tracking on/off by selecting the input option 310 in the exemplary screen display.

Referring again to Figs. 3A and 3B, the transducer 196 is configured to obtain ultrasound data along a plane within the anatomy. That is, the transducer 196 can focus the emitted energy 198 along the plane so that a cross-sectional image along the plane can be generated using the ultrasound data. As described herein, the processing system 130 and/or the ultrasound console 192 can determine velocity of blood flow and produce an associated velocity map along the imaging plane. Two exemplary planes 230a, 230b are illustrated in Figs. 3A and 3B. In some instances, the processing system 130 is configured to automatically select a suitable plane such that transducer 196 obtains the ultrasound data, including the velocity data, while aligned with the sensors 202, 204. In the illustrated embodiment, the emitted energy 198 is focused along the plane 230a, which is relatively close to the sensors 202, 204. Thus, the pressure data obtained by the sensor 202, 204, and the ultrasound data obtained by the transducer 196 are from the same area of the vessel 80.

The processing system 130 can rely on various factors to select imaging/velocity plane associated with the transducer 196. For example, the processing system 130 can utilize one or more of the geometry of the vessel 80, the location of the intravascular device 110 within the B-mode/flow image of the vessel 80, and/or the tracked location of the sensors 202, 204. The geometry of the vessel 80 can include the shape, diameter, area, volume, and/or other quantities based on the obtained imaging data. The vessel geometry can be utilized to ensure that the chosen imaging/velocity plane that does not include the vessel walls, for example. The location of the sensors 202, 204 may be extrapolated from the location of the intravascular device 110 within the B-mode/flow image of the vessel. Advantageously, by tracking the location of both sensors 202, 204, the imaging device 190 can visualize a full segment (e.g., distance D1) of the intravascular device 110 such that orientation of the transducer and/or the imaging plane is selected to ensure alignment with the fluid flow in the vessel 80. Furthermore, the sensors 202, 204 may be used to automatically estimate the Doppler angle in order to estimate the flow velocity in a more reliable fashion. The tracked location of the sensors 202, 204 provides a relatively more precise guidance allowing the velocity plane to be aligned in a desired manner. As shown in Fig. 4, the user may toggle automatic ultrasound plane selection on/off by selecting the input option 320 in the exemplary screen display. In some instances, as shown, the processing system 130 highlights the location of the selected imaging plane 230c in the ultrasound image 300.

In some embodiments, the vessel 80 in Fig. 1a, Fig. 1b, Fig. 1c, Fig. 3a, and Fig. 3b is a renal vessel consistent with the vessels 81 of Figure 2. The processing system 130 may determine the pulse wave velocity (PWV) in the renal artery. The processing system 130 may determine a renal denervation therapy recommendation based on the pulse wave velocity in a renal artery. For example, patients that are more likely or less likely to benefit therapeutically from renal denervation may be selected based on the PWV. In that regard, based at least on the PWV of blood in the renal vessel, the processing system 130 can perform patient stratification for renal denervation.

Fig. 4 illustrates an exemplary screen display 162 that is output by the processing system 130 to the display 160. The screen display 162 can include the ultrasound or B-mode image 300 depicting the vessel geometry. The velocity map 302 and/or the arrows 304 indicative the vector flow data can be overlaid on the image 300. The key or legend 306 correlates colors illustrated in the velocity map 302 with the corresponding velocities. The imaging plane 230c and/or the sensor indicators 308 may be selectively overlaid on the image 300. The screen display additionally includes input options 310, 320, 330 for selectively activating/deactivating sensor tracking, automatic ultrasound plane selection, and automatic ultrasound orientation, respectively. In an exemplary embodiment, the display 160 is touch-sensitive monitor such that the user input can be provided by touching and/or dragging the input options 310, 320, 330.

The screen display 162 also includes a region 340 illustrating the calculated pulse wave velocity. In the illustrated embodiment, the numerical value of the PWV is shown. In other instances, color coding and/or other graphics can be displayed together with the numerical value. For example, green, yellow, red, and/or other suitable colors and patterns may be used indicate the severity of the PWV.

A region 350 of the screen display 162 includes therapy recommendation determined by the processing system 130. As described herein the processing system 130 can use the calculated PWV within the renal artery to determine which patients are likely to benefit from renal denervation treatment. In the illustrated embodiment, the therapy recommendation is a textual indication. For example, "Poor," "Fair," "Good," and/or other suitable words may communicate the predicted benefit associated with therapy for the particular patient. In other instances, a numerical score, color coding, and/or other graphics representative of the therapy recommendation can be output to the display 160.

Fig. 5 is a flow diagram of a method 500 of determining pulse wave velocity. It is understood that the steps of method 500 may be performed in a different order than shown in Fig. 5, additional steps can be provided before, during, and after the steps, and/or some of the steps described can be replaced or eliminated in other embodiments. The steps of the method 500 can be performed by components of the intravascular system 100, 200, and 250 (Figs. 1A, 1B, and 1C).

At step 505, the method 500 includes positioning an intravascular device within the vessel. The intravascular device may include a pressure sensor. The vessel may be the renal artery. At step 510, the method 500 includes positioning an imaging device proximate to a body portion including the vessel. The imaging device may include an external ultrasound transducer. The steps 505 and 510 may be carried out by a clinician performing the diagnostic/therapeutic procedure and/or other user.

At step 515, the method 500 includes monitoring pressure associated with fluid within vessel. Step 515 may be performed using the intravascular device positioned within the vessel. At step 520, the method 500 includes monitoring velocity associated with the fluid within the vessel. Step 520 may be performed using the imaging device the imaging device positioned outside of a body portion including the vessel.

At step 525, the method 500 includes receiving pressure data associated with monitoring of pressure. For example, a processing system may receive the pressure data directly or indirectly from the intravascular device. In some instances, the method 500 includes processing the pressure data to obtain pressure measurements from within the vessel.

At step 530, the method 500 includes receiving velocity data associated with monitoring of velocity. For example, a processing system may receive the velocity data directly or indirectly from the imaging device. In some instances, the method 500 includes processing the imaging data obtained by the imaging device to extract the velocity data.

The method 500 can also include receiving lumen data associated with the vessel and obtained by the imaging device. In that regard, the imaging data can be processed to extract the lumen data. In some instances, the method 500 includes the processing system generating and outputting, to a display, a visual representation of the vessel and/or the velocity data. For example, an ultrasound or B-mode image including a velocity map may be displayed.

The method 500 can include synchronizing the collection of the pressure data and/or the velocity data. The method 500 can also include position the imaging device such that the velocity data and pressure data are obtained from the same area within the vessel.

In some embodiments, the ultrasound transducer of the imaging device obtains ultrasound data, including the velocity data, along a plane within the vessel. The method 500 may include the processing system determining the plane using vessel geometry and/or a location of the intravascular device. The processing system can determine the vessel geometry and/or the location of the intravascular device within the vessel based on the lumen data and/or the velocity data obtained by the imaging device. In other instances, the method 500 includes determining a location of the pressure sensor within the vessel. In such instances, the plane is determined using the vessel geometry and/or the location of the pressure sensor.

In some embodiments, the intravascular device includes the pressure sensor and a further pressure sensor. The method 500 may include determining locations of the pressure sensor and the further pressure sensor within the vessel. Additionally, the method 500 may include including obtaining velocity data while the imaging device is aligned with a direction of fluid flow based on the locations of the pressure sensor and the further pressure sensor. In that regard, the processing system may determine the direction of fluid flow based on the locations of the pressure sensors. Additionally, the angle of the direction of fluid flow with the imaging device may be used to automatically correct the determined velocity data. In some embodiments, the intravascular device is coupled to an imaging device holder. The method 500 may include outputting a control signal to an imaging device holder coupled to the imaging device to move the imaging device into alignment with the vessel. The processing system may generate and output the control signal based on identifying the location of the intravascular device and/or the pressure sensor within the vessel using the imaging data.

At step 535, the method 500 includes determine PWV of fluid within vessel based on pressure data and velocity data. In some embodiments, the PWV is determined as $PWV = \frac{1}{\rho}\frac{dP}{dU}$, where *dP* is a change in the pressure over a time interval, *dU* is a change in the velocity over the time interval, and $\rho$ is a density of the fluid within the vessel. In some embodiments, the PWV is determined by summation over the cardiac cycle as $PWV = \frac{1}{\rho}\sqrt{\frac{\sum dP^2}{\sum dU^2}}$ where *dP* is a change in the pressure over a time interval, *dU* is a change in the velocity over the time interval, and $\rho$ is a density of the fluid within the vessel. At step 540, the method 500 includes outputting a visual representation of the PWV. For example, the processing system can output display data associated with the visual representation to a display device.

At step 545, the method 500 includes determining a therapy recommendation based on the PWV. In that regard, the PWV within the renal artery may predict the effect of renal denervation in a patient. Accordingly, calculating the PWV facilitates selection of patients for whom this therapeutic procedure is likely beneficial. In some instances, the clinician determines the therapy recommendation based on the computed PWV and/or other patient data. In some embodiments, the processing system evaluates the PWV and/or other patient data to determine the therapy recommendation. In such instances, at step 550, the method 500 includes outputting a visual representation of the therapy recommendation. For example, the processing system can output display data associated with the graphical representation to a display device. The method 500 can additionally include classifying, based on the PWV, one or more patients into groups corresponding to respective degrees of predicted therapeutic benefit as a result of the renal denervation. The method 500 can also include the processing system outputting a graphical representation of the classifying step to the display device.

In some embodiments, the method 500 may be performed prior to performing a therapeutic procedure, e.g., prior to performing renal denervation. The method can determine the pulse wave velocity of a renal vessel that can be used for patient stratification and determining a renal denervation therapy recommendation. The method can be beneficial for patients with resistant hypertension. Renal sympathetic activity may worsen symptoms of hypertension, heart failure, and/or chronic renal failure. In particular, hypertension has been linked to increased sympathetic nervous system activity stimulated through any of four mechanisms, namely (1) increased vascular resistance, (2) increased cardiac rate, stroke volume and output, (3) vascular muscle defects, and/or (4) sodium retention and renin release by the kidney. As to this fourth mechanism in particular, stimulation of the renal sympathetic nervous system may affect renal function and maintenance of homeostasis. For example, an increase in efferent renal sympathetic nerve activity may cause increased renal vascular resistance, renin release, and sodium retention, all of which exacerbate hypertension.

Renal denervation, which affects both the electrical signals going into the kidneys (efferent sympathetic activity) and the electrical signals emanating from them (afferent sympathetic activity) may impact the mechanical and hormonal activities of the kidneys themselves, as well as the electrical activation of the rest of the SNS. Blocking efferent sympathetic activity to the kidney may alleviate hypertension and related cardiovascular diseases by reversing fluid and salt retention (augmenting natriuresis and diuresis), thereby lowering the fluid volume and mechanical load on the heart, and reducing inappropriate renin release, thereby halting the deleterious hormonal RAAS cascade before it starts.

By blocking afferent sympathetic activity from the kidney to the brain, renal denervation may lower the level of activation of the whole SNS. Thus, renal denervation may also decrease the electrical stimulation of other members of the sympathetic nervous system, such as the heart and blood vessels, thereby causing additional anti-hypertensive effects. In addition, blocking renal nerves may also have beneficial effects on organs damaged by chronic sympathetic over-activity, because it may lower the level of cytokines and hormones that may be harmful to the blood vessels, kidney, and heart.

Persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. The invention is defined by appended claims 1-13.

## Claims

**1.** An apparatus for pulse wave velocity (PWV) determination in a vessel (80), the apparatus comprising:

an intravascular device (110) sized and shaped for positioning within the vessel (80), the intravascular device (110) including:

a flexible elongate member (170) having a proximal portion (174) and a distal portion (172); and
a pressure sensor (202, 204) coupled to the distal portion (172) of the flexible elongate member (170), the pressure sensor (202, 204) configured to monitor a pressure of a fluid within the vessel (80);

an imaging device (190) configured to monitor a velocity of the fluid within the vessel (80), the imaging device (190) being configured to obtain imaging data from an external ultrasound transducer (196) configured for positioning proximate to a body portion (180) including the vessel (80); and
a processing system (130) in communication with the intravascular device (110) and the imaging device (190), the processing system (130) configured to:

synchronize the monitoring of the pressure within the vessel by the pressure sensor (202, 204) with the monitoring of the velocity within the vessel by the imaging device (190); and
determine the pulse wave velocity of the fluid within the vessel (80) based on the pressure and the velocity within the vessel.

**2.** The apparatus of claim 1, wherein the pulse wave velocity is determined as $\frac{1}{\rho}\frac{dP}{dU}$, or $\frac{1}{\rho}\sqrt{\frac{\sum dP^2}{\sum dU^2}}$, where *dP* is a change in the pressure over a time interval, *dU* is a change in the velocity over the time interval, and $\rho$ is a density of the fluid within the vessel (80).

**3.** The apparatus of claim 1,
wherein the ultrasound transducer (196) is configured to obtain ultrasound data along a plane within the vessel (80) to monitor the velocity of the fluid within the vessel (80), and
wherein the processing system (130) is configured to determine the plane using at least one of a vessel geometry or a location of the intravascular device (110) within the vessel (80) based on the lumen data and/or the monitoring the velocity of the fluid within the vessel (80).

**4.** The apparatus of claim 1, wherein the processing system (130) is further configured to output, to a display (160) in communication with the processing system (130), a visual representation of the pulse wave velocity.

**5.** The apparatus of claim 1, wherein the processing system (130) is further configured to:

determine a therapy recommendation based on the pulse wave velocity; and
output, to a display (160) in communication with the processing system (130), the therapy recommendation.

**6.** The apparatus of claim 5, wherein the vessel (80) comprises a renal artery, and wherein the processing system (130) is further configured to determine a renal denervation therapy recommendation based on the pulse wave velocity.

**7.** The apparatus of claim 1, wherein the vessel (80) comprises a renal artery, and wherein the processing system (130) is further configured to classify a patient based on a predicted therapeutic benefit of renal denervation using the pulse wave velocity.

**8.** A method of determining pulse wave velocity (PWV) in a vessel (80), comprising:

monitoring pressure associated with a fluid within the vessel (80), the monitoring pressure being performed using an intravascular device (110) including a pressure sensor (202, 204) and positioned within the vessel (80);
monitoring velocity associated with the fluid within the vessel (80), the monitoring velocity being performed using an imaging device (190) positioned outside of a body portion (180) including the vessel (80);
receiving pressure data associated with the monitoring of the pressure;
receiving velocity data associated with the monitoring of the velocity; and
determining the pulse wave velocity of the fluid within the vessel (80) based on the pressure data and the velocity data
wherein the monitoring of the pressure within the vessel (80) by the pressure sensor (202, 204) is synchronized by a processing system (130) with the monitoring of the velocity within the vessel (80) by the imaging device (190).

**9.** The method of claim 8, wherein the pulse wave velocity is determined as $\frac{1}{\rho}\frac{dP}{dU}$, or $\frac{1}{\rho}\sqrt{\frac{\sum dP^2}{\sum dU^2}}$, where *dP* is a change in the pressure over a time interval, *dU* is a change in the velocity over the time interval, and $\rho$ is a density of the fluid within the vessel (80).

**10.** The method of claim 8, wherein the vessel (80) is a renal artery.

**11.** The method of claim 8, wherein the imaging device (190) includes an external ultrasound transducer (196) configured for positioning proximate to the body portion (180) including the vessel (80).

**12.** The method of claim 11, wherein the receiving velocity data includes receiving ultrasound data obtained by the ultrasound transducer (196) along a plane within the vessel (80), and further comprising:
determining the plane using at least one of a vessel geometry or a location of a intravascular device (110) within the vessel (80) based on the lumen data and/or the velocity data.

**13.** The method of claim 8, further comprising:
outputting, to a display (160), a visual representation of the pulse wave velocity.

**Patentansprüche**

**1.** Vorrichtung zur Bestimmung der Pulswellengeschwindigkeit (PWV) in einem Blutgefäß (80), wobei die Vorrichtung umfasst:
eine intravaskuläre Vorrichtung (110), die zum Positionieren innerhalb des Blutgefäßes (80) bemessen und geformt ist, wobei die intravaskuläre Vorrichtung (110) beinhaltet:

ein flexibles längliches Element (170) mit einem proximalen Abschnitt (174) und einem distalen Abschnitt (172); und
einen Drucksensor (202, 204), der mit dem distalen Abschnitt (172) des flexiblen länglichen Elements (170) gekoppelt ist, wobei der Drucksensor (202, 204) konfiguriert ist, um einen Druck einer Flüssigkeit innerhalb des Blutgefäßes (80) zu überwachen;
eine Bildgebungsvorrichtung (190), die konfiguriert ist, um eine Geschwindigkeit der Flüssigkeit innerhalb des Blutgefäßes (80) zu überwachen, wobei die Bildgebungsvorrichtung (190) konfiguriert ist, um Bildgebungsdaten von einem externen Ultraschallwandler (196) zu erhalten, der zur Positionierung in der Nähe eines Körperabschnitts (180), der das Blutgefäß (80) beinhaltet, konfiguriert ist; und
ein Verarbeitungssystem (130) in Verbindung mit der intravaskulären Vorrichtung (110) und der Bildgebungsvorrichtung (190), wobei das Verarbeitungssystem (130) konfiguriert ist, um:

die Überwachung des Drucks innerhalb des Blutgefäßes durch den Drucksensor (202, 204) mit der Überwachung der Geschwindigkeit innerhalb des Blutgefäßes durch die Bildgebungsvorrichtung (190) zu synchronisieren; und
die Pulswellengeschwindigkeit der Flüssigkeit innerhalb des Blutgefäßes (80) basierend auf dem Druck und der Geschwindigkeit innerhalb des Blutgefäßes zu bestimmen.

**2.** Vorrichtung nach Anspruch 1, wobei die Pulswellengeschwindigkeit bestimmt wird als $\frac{1}{\rho}\frac{dP}{dU}$ oder $\frac{1}{\rho}\sqrt{\frac{\sum dP^2}{\Sigma dU^2}}$, wobei

dP eine Änderung des Drucks über ein Zeitintervall ist, dU eine Änderung der Geschwindigkeit über das Zeitintervall ist und p eine Dichte der Flüssigkeit innerhalb des Blutgefäßes (80) ist.

**3.** Vorrichtung nach Anspruch 1,
wobei der Ultraschallwandler (196) konfiguriert ist, um Ultraschalldaten entlang einer Ebene innerhalb des Blutgefäßes (80) zu erhalten, um die Geschwindigkeit der Flüssigkeit innerhalb des Blutgefäßes (80) zu überwachen, und wobei das Verarbeitungssystem (130) konfiguriert ist, um die Ebene unter Verwendung mindestens einer der Blutgefäßgeometrien oder einer Lage der intravaskulären Vorrichtung (110) innerhalb des Blutgefäßes (80) basierend auf den Lumendaten und/oder der Überwachung der Geschwindigkeit der Flüssigkeit innerhalb des Blutgefäßes (80) zu bestimmen.

**4.** Vorrichtung nach Anspruch 1, wobei das Verarbeitungssystem (130) weiter konfiguriert ist, um an eine Anzeige (160) in Verbindung mit dem
Verarbeitungssystem (130) eine visuelle Darstellung der Pulswellengeschwindigkeit auszugeben.

**5.** Vorrichtung nach Anspruch 1, wobei das Verarbeitungssystem (130) weiter konfiguriert ist, um:

eine Therapieempfehlung basierend auf der Pulswellengeschwindigkeit zu bestimmen; und
die Therapieempfehlung an eine Anzeige (160) in Verbindung mit dem Verarbeitungssystem (130) auszugeben.

**6.** Vorrichtung nach Anspruch 5, wobei das Gefäß (80) eine Nierenarterie umfasst, und wobei das Verarbeitungssystem (130) weiter konfiguriert ist, um eine Empfehlung für eine Nierendenervationstherapie basierend auf der Pulswellengeschwindigkeit zu bestimmen.

**7.** Vorrichtung nach Anspruch 1, wobei das Blutgefäß (80) eine Nierenarterie umfasst, und wobei das Verarbeitungssystem (130) weiter konfiguriert ist, um einen Patienten basierend auf einem vorhergesagten therapeutischen Nutzen der Nierendenervation unter Verwendung der Pulswellengeschwindigkeit zu klassifizieren.

**8.** Verfahren zum Bestimmen der Pulswellengeschwindigkeit (PWV) in einem Blutgefäß (80), umfassend:

Überwachen des Drucks, der einer Flüssigkeit innerhalb des Blutgefäßes (80) zugeordnet ist, wobei der Überprüfungsdruck unter Verwendung einer intravaskulären Vorrichtung (110) mit einem Drucksensor (202, 204) durchgeführt wird, die innerhalb des Blutgefäßes (80) positioniert ist;
Überwachen der Geschwindigkeit, die der Flüssigkeit innerhalb des Blutgefäßes (80) zugeordnet ist, wobei die Überprüfungsgeschwindigkeit unter Verwendung einer Bildgebungsvorrichtung (190) durchgeführt wird, die außerhalb eines Körperteils (180), der das Blutgefäß (80) beinhaltet, angeordnet ist;
Empfangen von Druckdaten, die der Überwachung des Drucks zugeordnet sind;
Empfangen von Geschwindigkeitsdaten, die der Überwachung der Geschwindigkeit zugeordnet sind; und
Bestimmen der Pulswellengeschwindigkeit der Flüssigkeit innerhalb des Blutgefäßes (80) basierend auf den Druckdaten und den Geschwindigkeitsdaten,
wobei die Überwachung des Drucks innerhalb des Blutgefäßes (80) durch den Drucksensor (202, 204) durch ein Verarbeitungssystem (130) mit der Überwachung der Geschwindigkeit innerhalb des Blutgefäßes (80) durch die Bildgebungsvorrichtung (190) synchronisiert wird.

**9.** Verfahren nach Anspruch 8, wobei die Pulswellengeschwindigkeit bestimmt wird als $\frac{1}{\rho}\frac{dP}{dU}$, oder $\frac{1}{\rho}\sqrt{\frac{\sum dP^2}{\sum dU^2}}$, wobei dP eine Änderung des Drucks über ein Zeitintervall ist, dU eine Änderung der Geschwindigkeit über das Zeitintervall ist und p eine Dichte der Flüssigkeit innerhalb des Blutgefäßes (80) ist.

**10.** Verfahren nach Anspruch 8, wobei das Blutgefäß (80) eine Nierenarterie ist.

**11.** Verfahren nach Anspruch 8, wobei die Bildgebungsvorrichtung (190) einen externen Ultraschallwandler (196) beinhaltet, der zur Positionierung in der Nähe des Körperabschnitts (180), der das Blutgefäß (80) beinhaltet, konfiguriert ist.

**12.** Verfahren nach Anspruch 11, wobei das Empfangen von Geschwindigkeitsdaten das Empfangen von Ultraschalldaten beinhaltet, die durch den Ultraschallwandler (196) entlang einer Ebene innerhalb des Blutgefäßes (80) erhalten wurden, und weiter umfasst:
Bestimmen der Ebene unter Verwendung mindestens einer Blutgefäßgeometrie oder einer Lage einer intravaskulären Vorrichtung (110) innerhalb des Blutgefäßes (80) basierend auf den Lumendaten und/oder den Geschwindigkeitsdaten.

**13.** Verfahren nach Anspruch 8, weiter umfassend:
Ausgeben einer visuellen Darstellung der Pulswellengeschwindigkeit an eine Anzeige (160).

## Revendications

**1.** Appareil de détermination de la vitesse des ondes pulsatiles (PWV) dans un vaisseau (80), l'appareil comprenant : un dispositif intravasculaire (110) dimensionné et façonné pour être positionné à l'intérieur du vaisseau (80), le dispositif intravasculaire (110) incluant :

un élément allongé souple (170) ayant une partie proximale (174) et une partie distale (172) ; et
un capteur de pression (202, 204) couplé à la partie distale (172) de l'élément allongé souple (170), le capteur de pression (202, 204) configuré pour surveiller une pression d'un fluide à l'intérieur du vaisseau (80) ;

un dispositif d'imagerie (190) configuré pour surveiller une vitesse du fluide à l'intérieur du vaisseau (80), le dispositif d'imagerie (190) étant configuré pour obtenir des données d'imagerie à partir d'un transducteur ultrasonore externe (196) configuré pour être positionné à proximité d'une partie corps (180) incluant le vaisseau (80) ; et
un système de traitement (130) en communication avec le dispositif intravasculaire (110) et le dispositif d'imagerie (190), le système de traitement (130) configuré pour :

synchroniser la surveillance de la pression à l'intérieur du vaisseau par le capteur de pression (202, 204) avec la surveillance de la vitesse à l'intérieur du vaisseau par le dispositif d'imagerie (190) ; et
déterminer la vitesse des ondes pulsatiles du fluide à l'intérieur du vaisseau (80) sur la base de la pression et de la vitesse à l'intérieur du vaisseau.

**2.** Appareil selon la revendication 1, dans lequel la vitesse des ondes pulsatiles est déterminée comme étant $\frac{1}{\rho}\frac{dP}{dU}$

$\frac{1}{\rho}\sqrt{\frac{\sum dP^2}{\sum dU^2}}$, où *dP* est un changement de la pression sur un intervalle de temps, *dU* est un changement de la vitesse sur l'intervalle de temps, et $\rho$ est une masse volumique du fluide à l'intérieur du vaisseau (80).

**3.** Appareil selon la revendication 1,
dans lequel le transducteur ultrasonore (196) est configuré pour obtenir des données ultrasonores le long d'un plan à l'intérieur du vaisseau (80) pour surveiller la vitesse du fluide à l'intérieur du vaisseau (80), et
dans lequel le système de traitement (130) est configuré pour déterminer le plan en utilisant au moins l'un d'une géométrie du vaisseau ou d'un emplacement du dispositif intravasculaire (110) à l'intérieur du vaisseau (80) sur la base des données de lumière et/ou de la surveillance de la vitesse du fluide à l'intérieur du vaisseau (80).

**4.** Appareil selon la revendication 1, dans lequel le système de traitement (130) est en outre configuré pour délivrer en sortie, à un écran d'affichage (160) en communication avec le système de traitement (130), une représentation visuelle de la vitesse des ondes pulsatiles.

**5.** Appareil selon la revendication 1, dans lequel le système de traitement (130) est en outre configuré pour :

déterminer une recommandation thérapeutique sur la base de la vitesse des ondes pulsatiles ; et
délivrer en sortie, à un écran d'affichage (160) en communication avec le système de traitement (130), la recommandation thérapeutique.

**6.** Appareil selon la revendication 5, dans lequel le vaisseau (80) comprend une artère rénale, et dans lequel le système de traitement (130) est en outre configuré pour déterminer une recommandation thérapeutique de dénervation rénale sur la base de la vitesse des ondes pulsatiles.

**7.** Appareil selon la revendication 1, dans lequel le vaisseau (80) comprend une artère rénale, et dans lequel le système de traitement (130) est en outre configuré pour classer un patient sur la base d'un bénéfice thérapeutique prédit de la dénervation rénale en utilisant la vitesse des ondes pulsatiles.

**8.** Procédé de détermination de la vitesse des ondes pulsatiles (PWV) dans un vaisseau (80), comprenant :

la surveillance de la pression associée à un fluide à l'intérieur du vaisseau (80), la surveillance de la pression étant effectuée en utilisant un dispositif intravasculaire (110) incluant un capteur de pression (202, 204) et positionné à l'intérieur du vaisseau (80) ;
la surveillance de la vitesse associée au fluide à l'intérieur du vaisseau (80), la surveillance de la vitesse étant effectuée en utilisant un dispositif d'imagerie (190) positionné à l'extérieur d'une partie corps (180) incluant le vaisseau (80) ;
la réception de données de pression associées à la surveillance de la pression ;
la réception de données de vitesse associées à la surveillance de la vitesse ; et
la détermination de la vitesse des ondes pulsatiles du fluide à l'intérieur du vaisseau (80) sur la base des données de pression et des données de vitesse

dans lequel la surveillance de la pression à l'intérieur du vaisseau (80) par le capteur de pression (202, 204) est synchronisée par un système de traitement (130) avec la surveillance de la vitesse à l'intérieur du vaisseau (80) par le dispositif d'imagerie (190).

**9.** Procédé selon la revendication 8, dans lequel la vitesse des ondes pulsatiles est déterminée comme étant $\frac{1}{\rho}\frac{dP}{dU}$ ou $\frac{1}{\rho}\sqrt{\frac{\sum dP^2}{\sum dU^2}}$, où *dP* est un changement de la pression sur un intervalle de temps, *dU* est un changement de la vitesse sur l'intervalle de temps, et $\rho$ *est* une masse volumique du fluide à l'intérieur du vaisseau (80).

**10.** Procédé selon la revendication 8, dans lequel le vaisseau (80) est une artère rénale.

**11.** Procédé selon la revendication 8, dans lequel le dispositif d'imagerie (190) inclut un transducteur ultrasonore externe (196) configuré pour être positionné à proximité de la partie corps (180) incluant le vaisseau (80).

**12.** Procédé selon la revendication 11, dans lequel la réception des données de vitesse inclut la réception des données ultrasonores obtenues par le transducteur ultrasonore (196) le long d'un plan à l'intérieur du vaisseau (80), et comprenant en outre :
la détermination du plan en utilisant au moins l'un d'une géométrie du vaisseau ou d'un emplacement d'un dispositif intravasculaire (110) à l'intérieur du vaisseau (80) sur la base des données de lumière et/ou des données de vitesse.

**13.** Procédé selon la revendication 8, comprenant en outre :
la délivrance en sortie, à un écran d'affichage (160), d'une représentation visuelle de la vitesse des ondes pulsatiles.

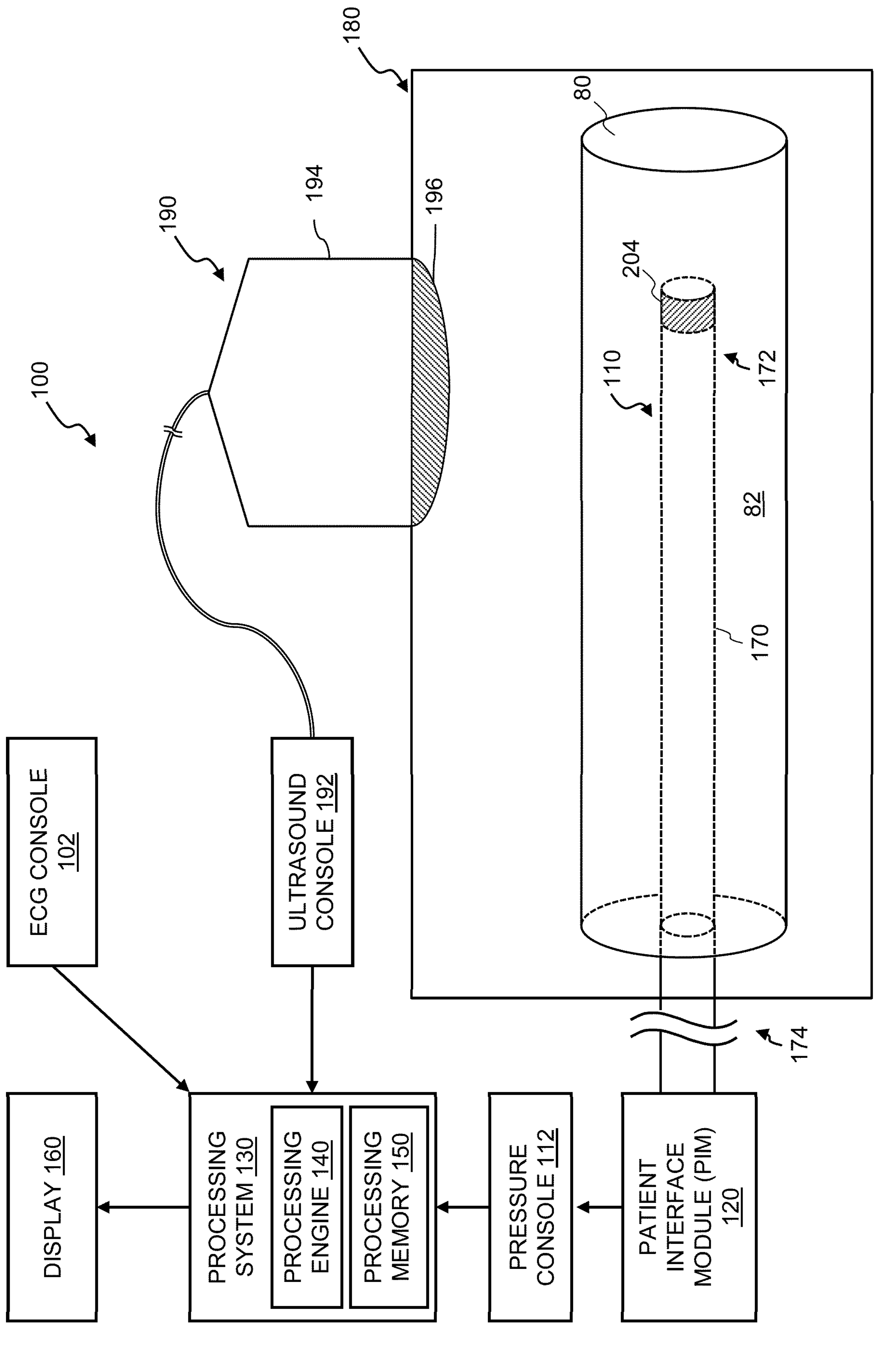
100
190
194
196
180
80
204
110
172
82
170
174
ECG CONSOLE 102
ULTRASOUND CONSOLE 192
DISPLAY 160
PROCESSING SYSTEM 130
PROCESSING ENGINE 140
PROCESSING MEMORY 150
PRESSURE CONSOLE 112
PATIENT INTERFACE MODULE (PIM) 120

Fig. 1A

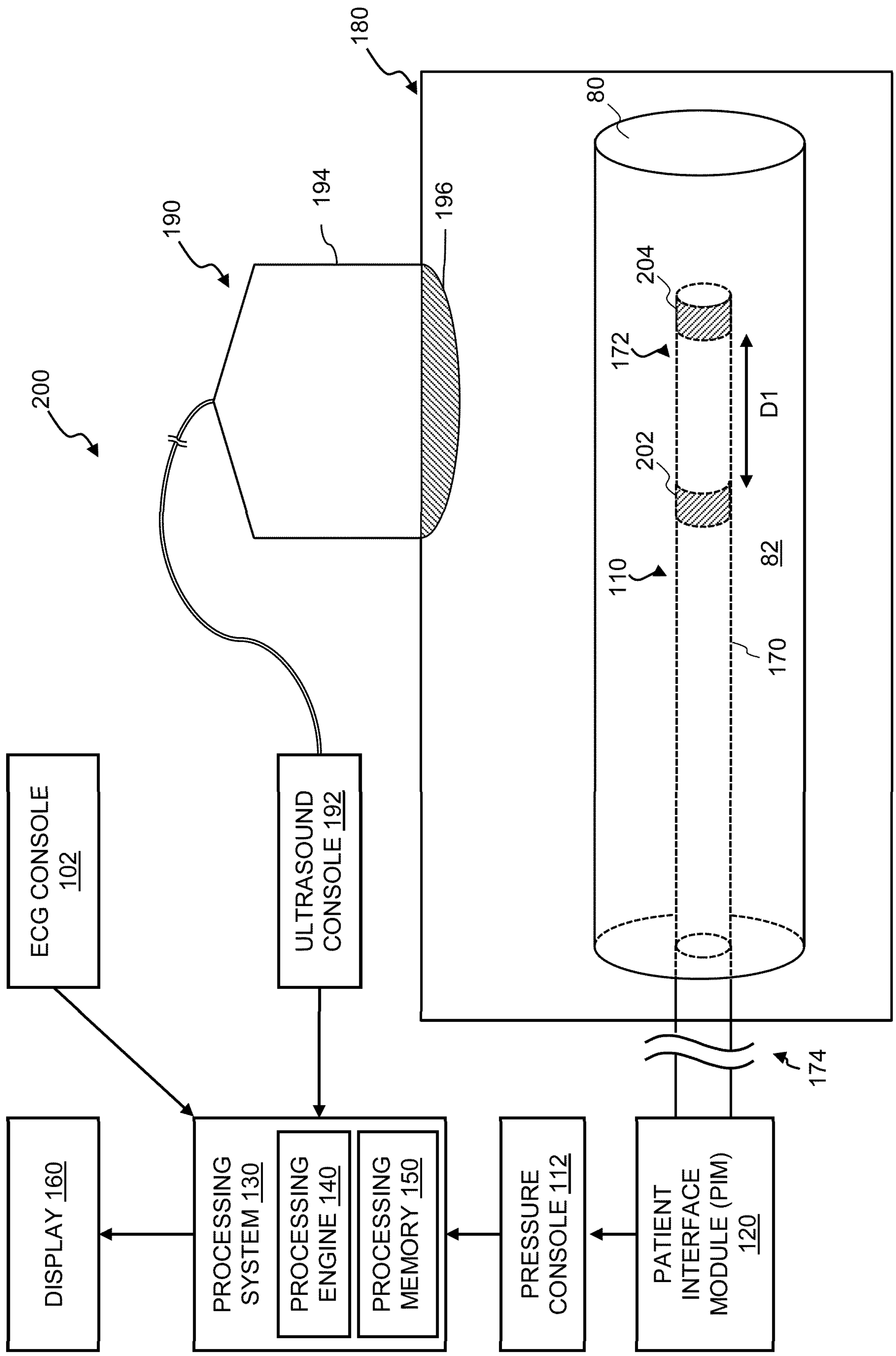
200
190
194
196
180
80
204
172
D1
202
110
82
170
174
ECG CONSOLE 102
ULTRASOUND CONSOLE 192
DISPLAY 160
PROCESSING SYSTEM 130
PROCESSING ENGINE 140
PROCESSING MEMORY 150
PRESSURE CONSOLE 112
PATIENT INTERFACE MODULE (PIM) 120

Fig. 1B

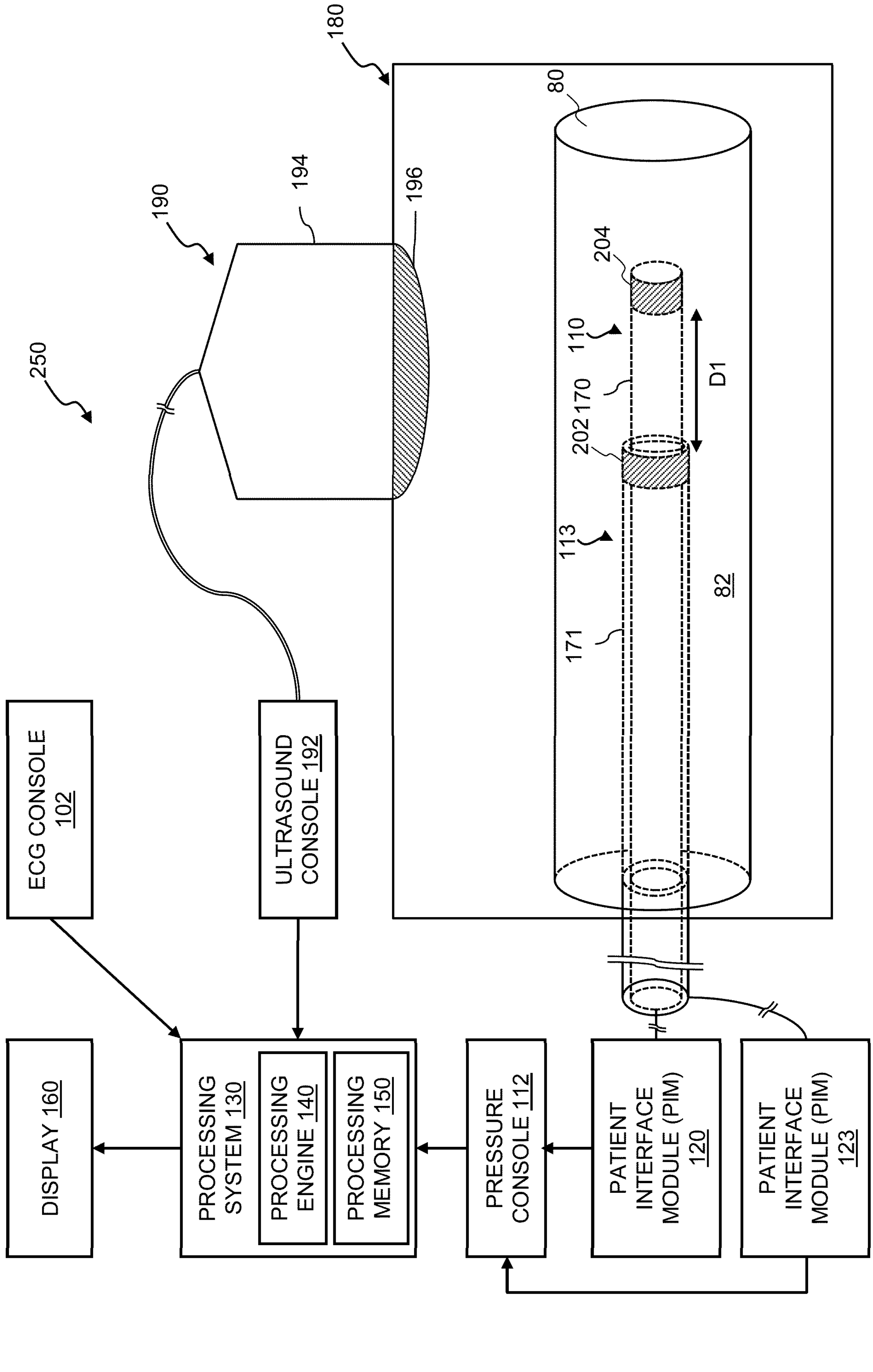
250
190
194
196
180
80
204
110
D1
202
170
113
82
171
ECG CONSOLE 102
ULTRASOUND CONSOLE 192
DISPLAY 160
PROCESSING SYSTEM 130
PROCESSING ENGINE 140
PROCESSING MEMORY 150
PRESSURE CONSOLE 112
PATIENT INTERFACE MODULE (PIM) 120
PATIENT INTERFACE MODULE (PIM) 123

Fig. 1C

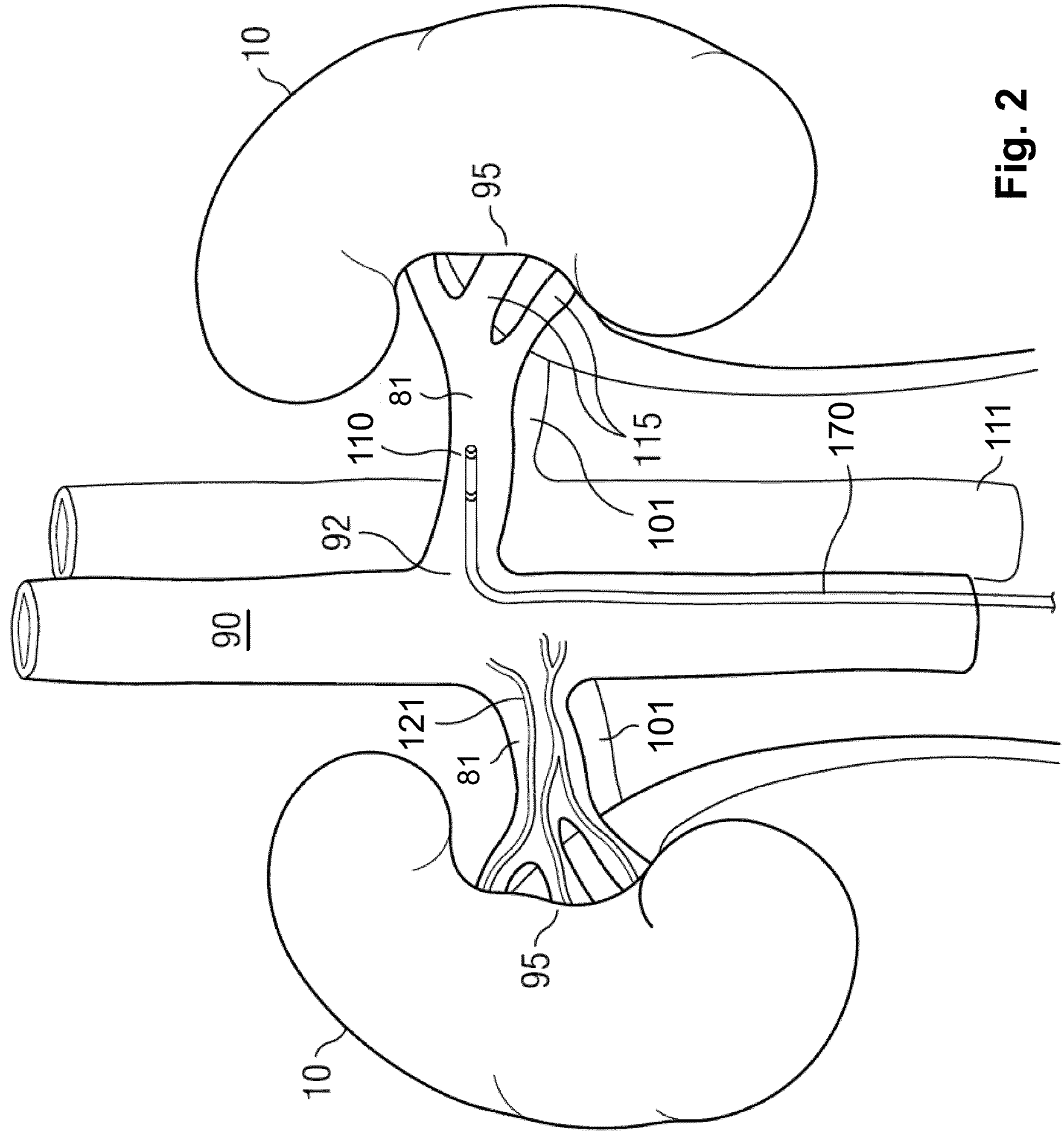


Fig. 2

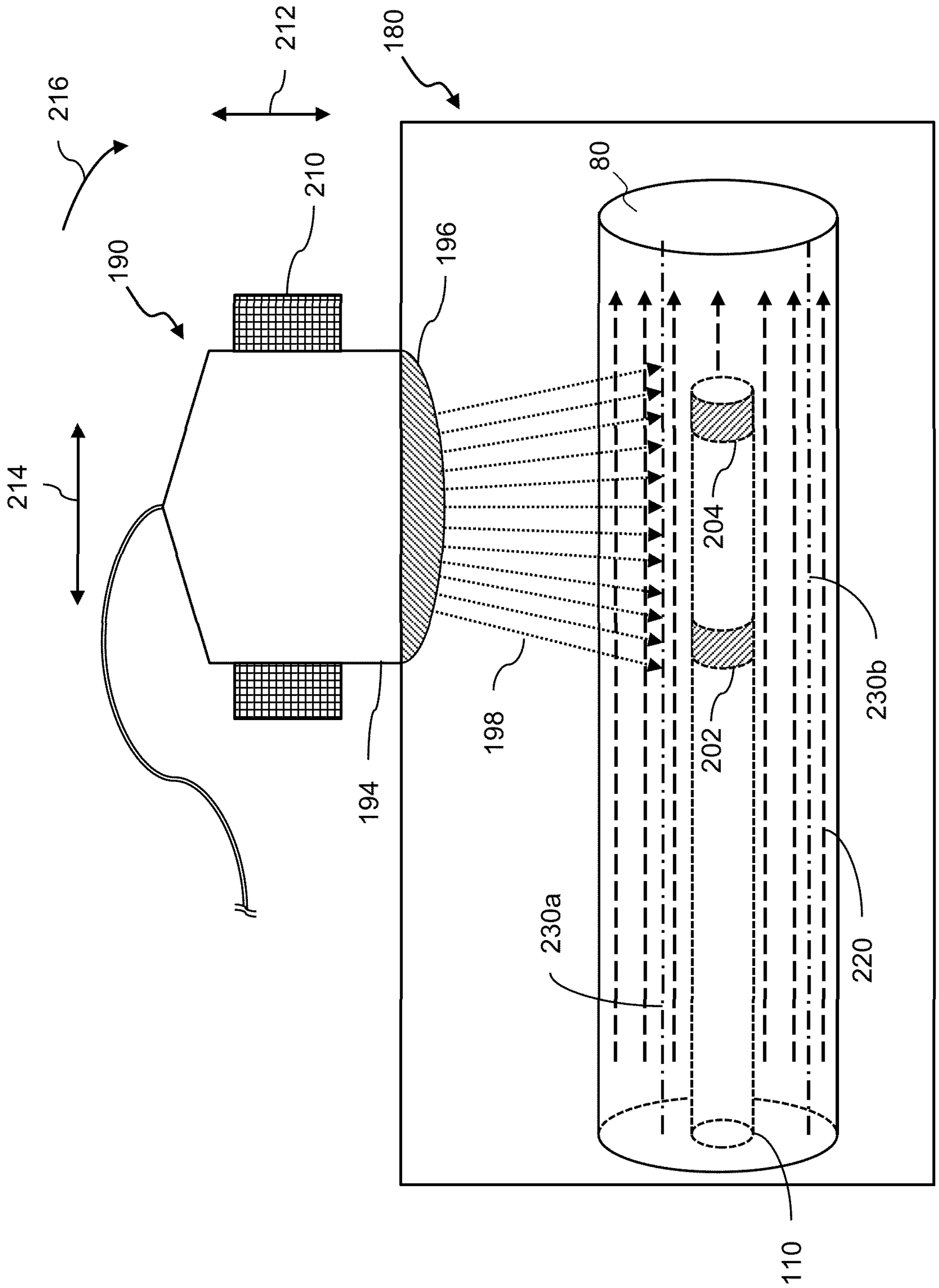


Fig. 3A

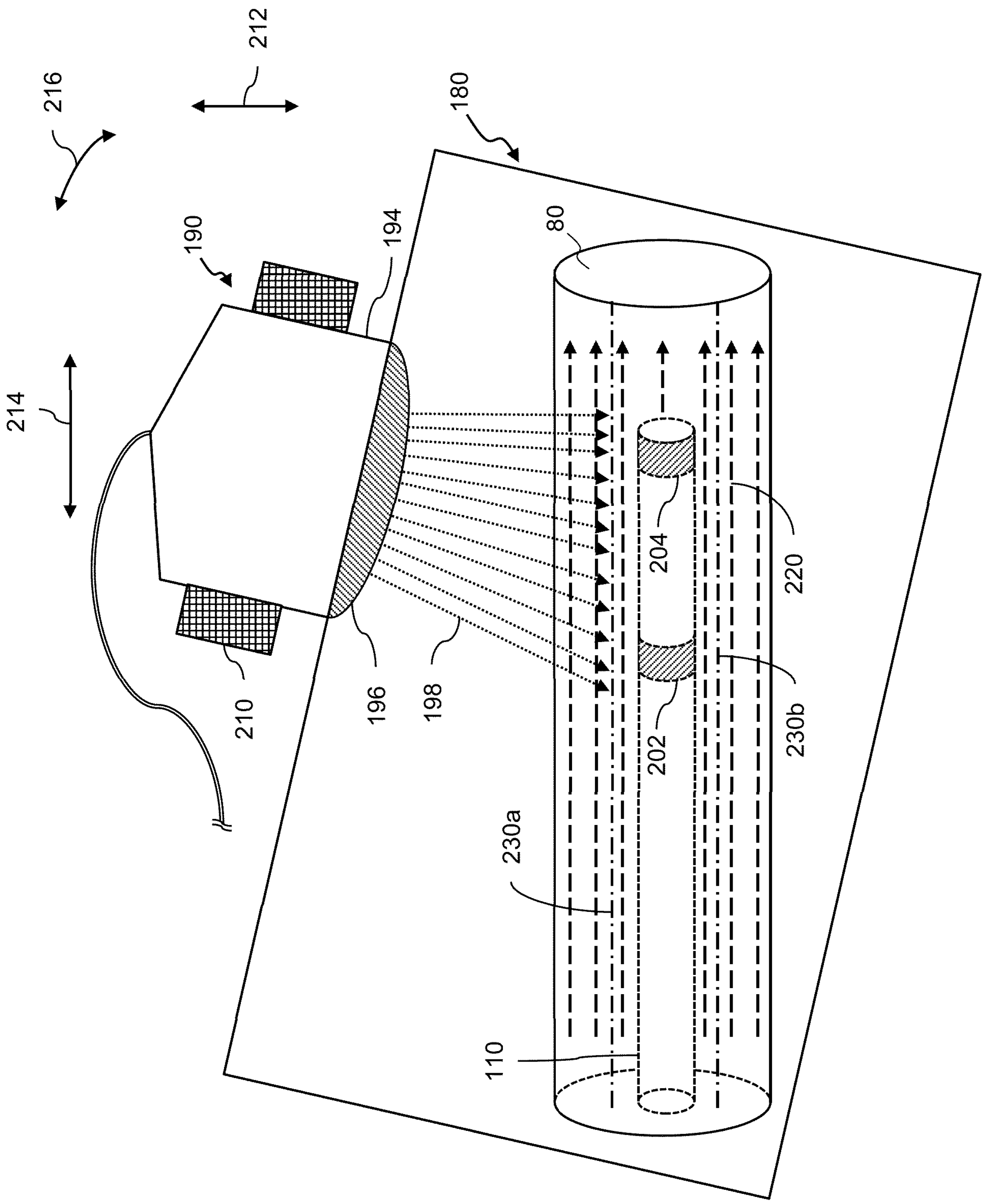


Fig. 3B

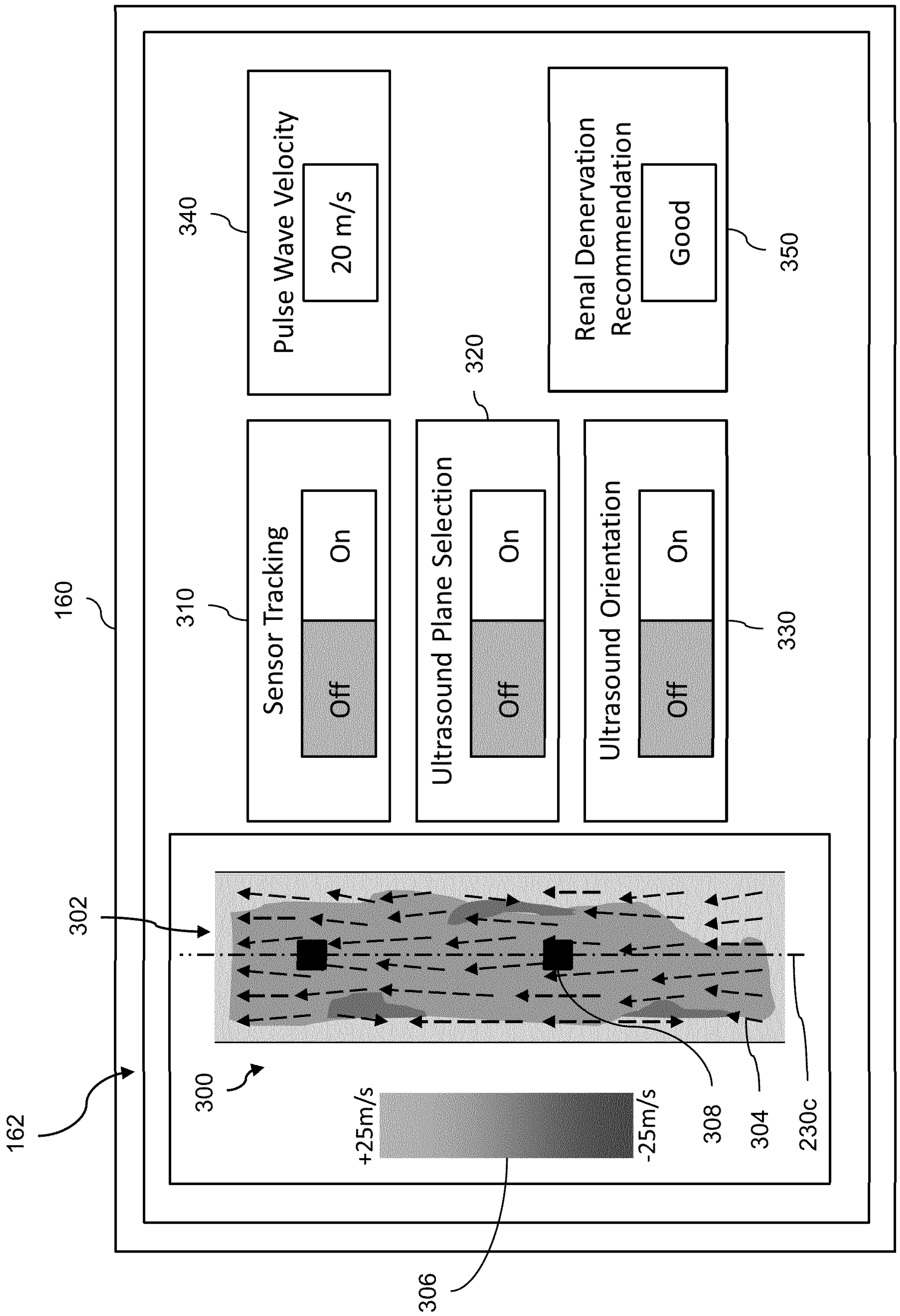
160
162
302
300
+25m/s
-25m/s
308
304
230c
306
310
Sensor Tracking
Off
On
320
Ultrasound Plane Selection
Off
On
330
Ultrasound Orientation
Off
On
340
Pulse Wave Velocity
20 m/s
350
Renal Denervation Recommendation
Good

Fig. 4

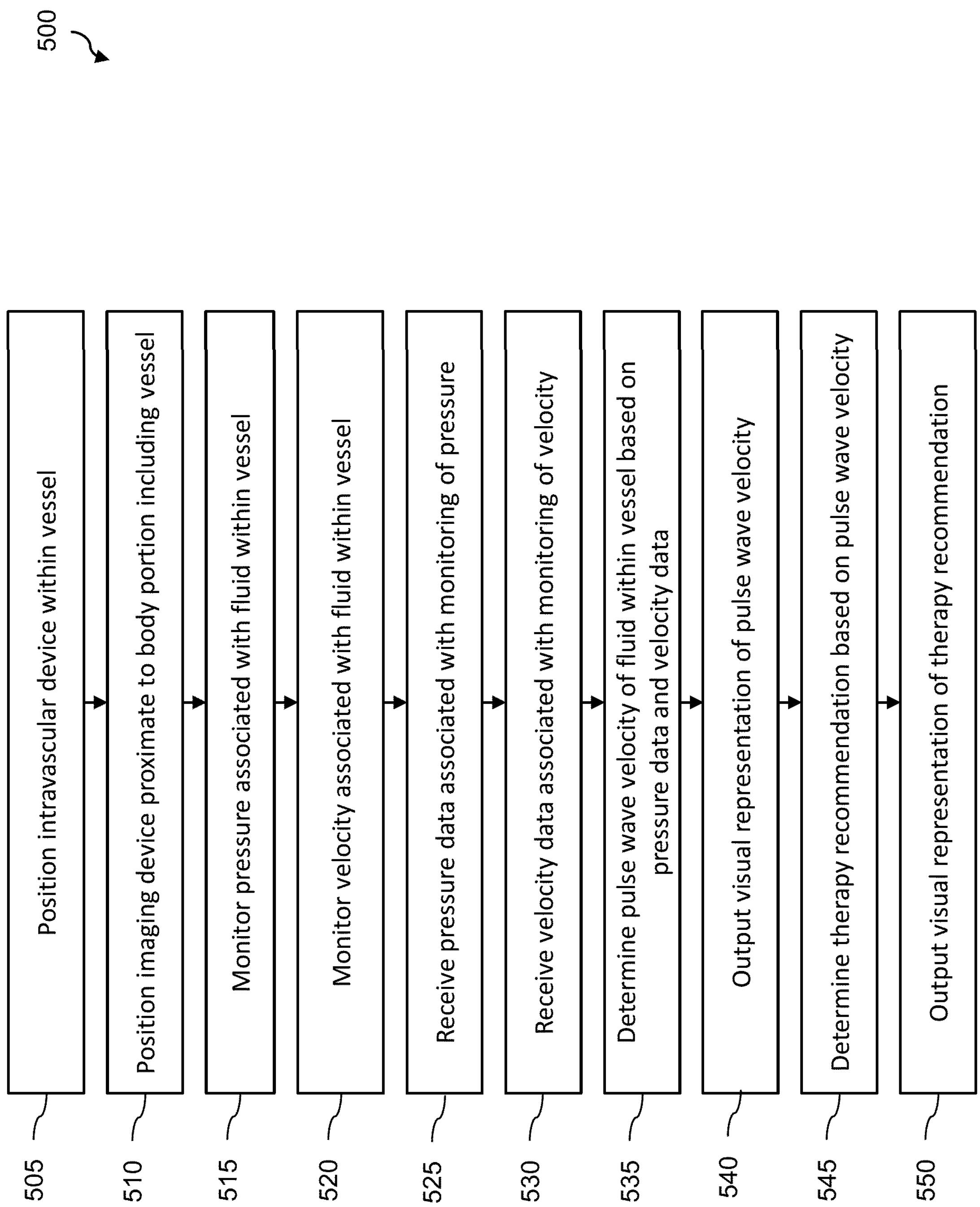
500
505
Position intravascular device within vessel
510
Position imaging device proximate to body portion including vessel
515
Monitor pressure associated with fluid within vessel
520
Monitor velocity associated with fluid within vessel
525
Receive pressure data associated with monitoring of pressure
530
Receive velocity data associated with monitoring of velocity
535
Determine pulse wave velocity of fluid within vessel based on pressure data and velocity data
540
Output visual representation of pulse wave velocity
545
Determine therapy recommendation based on pulse wave velocity
550
Output visual representation of therapy recommendation

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0055579 A2
- US 20090270695 A1
- WO 2011138698A1 A